(19) Europäisches Patentamt
European Patent Office
Office européen des brevets

(11) Publication number: **0 300 797 B1**

# (12) EUROPEAN PATENT SPECIFICATION

(45) Date of publication of patent specification: **04.10.95**

(51) Int. Cl.[6]: **C07D 493/08**, C07D 495/08, C07D 497/08, C07D 305/04, A01N 43/90, //(C07D493/08, 311:00,319:00),(C07D493/08, 319:00,319:00),(C07D495/08, 339:00,339:00)

(21) Application number: **88306718.3**

(22) Date of filing: **21.07.88**

Divisional application 93100641.5 filed on 21/07/88.

The file contains technical information submitted after the application was filed and not included in this specification

## (54) Pesticidal compounds.

(30) Priority: **22.07.87 GB 8717274**
**07.10.87 GB 8723488**
**26.04.88 GB 8809851**

(43) Date of publication of application:
**25.01.89 Bulletin 89/04**

(45) Publication of the grant of the patent:
**04.10.95 Bulletin 95/40**

(84) Designated Contracting States:
**AT BE CH DE ES FR GB GR IT LI LU NL SE**

(56) References cited:
**EP-A- 0 152 229**
**EP-A- 0 211 598**
**EP-A- 0 216 624**
**EP-A- 0 216 625**
**US-A- 3 686 224**

**J.Agric.Food Chem.33,976(1985)**

**Mol.Pharmacol.28,246(1985)**

**ACS Symp.356,71(1987)**

(73) Proprietor: **ROUSSEL UCLAF**
**102 Route de Noisy**
**F-93230 Romainville (FR)**

(72) Inventor: **Weston, John Bernard**
**The Wellcome Research Laboratories**
**Ravens Lane**
**Berkhamsted**
**Hertfordshire (GB)**
Inventor: **Larkin, John Patrick**
**The Wellcome Research Laboratories**
**Ravens Lane**
**Berkhamsted**
**Hertfordshire (GB)**
Inventor: **Smith, Ian Harold**
**The Wellcome Research Laboratories**
**Ravens Lane**
**Berkhamsted**
**Hertfordshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 300 797 B1

Rank Xerox (UK) Business Services
(3.10/3.09/3.3.3)

(74) Representative: **Vieillefosse, Jean-Claude et al**
**ROUSSEL UCLAF**
**Département des Brevets**
**102, Route de Noisy**
**F-93235 Romainville (FR)**

## Description

The present invention relates to novel chemical compounds having pesticidal activity, to methods for their preparation, to compositions containing them and to their use in the control of pests. More particularly the invention relates to a class of heterobicycloalkanes.

The use of certain pesticidally active 2,6,7-trioxabicyclo[2.2.2]octanes is disclosed in European Patent Applications Nos. 152229, 211598, 216625, 216624, US Patent 3686224 and J.Agric.Food Chem. 33, 976 (1985). It has now been discovered that derivatives of these compounds have interesting pesticidal activity.

Accordingly, the present invention provides a compound of the formula (I):

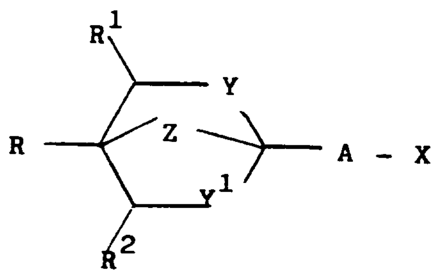

(I)

wherein R is a $C_{2-10}$ non-aromatic hydrocarbyl group optionally substituted by, or methyl substituted by cyano, halo, $C_{1-4}$ alkoxy optionally substituted by halo, or a group $S(O)_mR^3$ where $R^3$ is $C_{1-4}$ alkyl optionally substituted by halo and m is 0, 1 or 2, or R is phenyl optionally substituted by $C_{1-4}$ alkoxy, $C_{1-3}$ alkyl, $C_{2-4}$ alkynyl, halo, $C_{1-4}$ haloalkyl, cyano or a group $S(O)_mR^3$ as defined hereinbefore;

$R^1$ and $R^2$ may be the same or different, and each is hydrogen,

methyl, $CF_3$ or cyano

A-X is $(CH_2)_4$-C≡C-H;

Y and $Y^1$ are the same or different and are each selected from oxygen and $S(O)_{n'}$ where n' is 0, 1 or 2; and Z is $CH_2CH_2$, $CH_2O$ or $CH_2S(O)_{n''}$, wherein n'' is 0, 1 or 2;

In the definition of Z, the first mentioned atom is adjacent to the 4-position of the bicyclic ring system.

When R is an alkyl, alkenyl or alkynyl, cycloalkyl or cycloalkenyl group this preferably contains up to 6 carbon atoms. Conveniently, when R is substituted there are up to seven substituents when the substituent is fluoro, three substituents when the substituents are chloro or bromo or one substituent when this is other than halo.

Suitably, R is an aliphatic or alicyclic group containing between 2 and 8 carbon atoms or phenyl each optionally substituted by cyano, one to seven halo atoms, $C_{1-4}$ alkoxy or a group $S(O)_m R^4$ as hereinbefore defined. Most suitably R is propyl, butyl, pentyl, $C_{2-5}$ alkenyl or alkynyl, cyclopropylmethyl, $C_{3-7}$ cycloalkyl or phenyl each optionally substituted by fluoro, chloro or bromo, for example, n-propyl, n-butyl, i-butyl, sec-butyl t-butyl, prop-2-enyl, 2-methylprop-2-enyl, but-3-enyl, phenyl, cyclopentyl or cyclohexyl. Preferably R is n-propyl, n-butyl, i-butyl, t-butyl or phenyl.

Preferably $R^2$ is hydrogen.

Preferably Z is $CH_2S$ or $CH_2O$.

Suitably Y and $Y^1$ are both selected from oxygen or sulphur.

The compounds of the formula (I) may exist in a number of isomeric forms. The present invention provides individual isomers of compounds of the formula (I) and mixtures thereof. The present invention also encompasses compounds of the formula (I) containing radioisotopes, particularly those in which one carbon atom is $C^{14}$ or one to three hydrogen atoms are replaced by tritium.

Preferred compounds of the present invention include:

1-(Hex-5-ynyl)-4-propyl-2,6,7-trioxabicyclo[2.2.2]octane
1-(Hex-5-ynyl)-4-propyl-3-trifluoromethyl-2,6,7-trioxabicyclo[2.2.2]octane
1-(Hex-5-ynyl)-4-propyl-2,6,7-trioxabicyclo[2.2.2]octane-3-carbonitrile
4-(Cyclohexyl)-1-(hex-5-ynyl)-2,6,7-trioxabicyclo[2.2.2]octane
4-t-Butyl-1-(hex-5-ynyl)-2,6,7-trioxabicyclo[2.2.2]octane
4-t-Butyl-1-(6-trimethylsilylhex-5-ynyl)-2,6,7-trioxabicyclo[2.2.2]octane
4-t-Butyl-1-(hex-5-ynyl)-2,6-dioxa-7-thiabicyclo[2.2.2]octane
1-(Hex-5-ynyl)-4-propyl-2-oxa-6,7-dithiabicyclo[2.2.2]octane

1-(Hex-5-ynyl)-4-propyl-2,6,7-trithiabicyclo[2.2.2]octane
4-Butyl-1-(hex-5-ynyl)-2,6,7-trioxabicyclo[2.2.2]octane-3-carbonitrile
1-(Hex-5-ynyl)-4-(2-methylprop-2-enyl)-2,6,7-trioxabicyclo[2.2.2]octane-3-carbonitrile
1-(Hex-5-ynyl)-4-(prop-2-enyl)-2,6,7-trioxabicyclo[2.2.2]octane-3-carbonitrile
4-(But-3-enyl)-1-(hex-5-ynyl)-2,6,7-trioxabicyclo[2.2.2]octane-3-carbonitrile
4-Butyl-1-(hex-5-ynyl)-2-oxa-6,7-dithiabicyclo[2.2.2]octane
4-t-Butyl-1-(hex-5-ynyl)-2-oxa-6,7-dithiabicyclo[2.2.2]octane
4-t-Butyl-1-(hex-5-ynyl)-2,6,7-trioxabicyclo[2.2.2]octane-3-carbonitrile
1-(Hex-5-ynyl)-4-isobutyl-2,6,7-trioxabicyclo[2.2.2]octane-3-carbonit rile
4-Ethoxymethyl-1-(hex-5-ynyl)-2,6,7-trioxabicyclo[2.2.2]octane-3-carbonitrile
1-(Hex-5-ynyl)-4-phenyl-2,6,7-trioxabicyclo[2.2.2]octane
1-(Hex-5-ynyl)-4-isobutyl-2-oxa-6,7-dithiabicyclo[2.2.2]octane
1-(Hex-5-ynyl)-3-methyl-4-propyl-2-oxa-6,7-dithiabicyclo[2.2.2]octane
1-(Hex-5-ynyl)-4-isobutyl-2,6,7-trithiabicyclo[2.2.2]octane
1-(Hex-5-ynyl)-4-phenyl-2-oxa-6,7-dithiabicyclo[2.2.2]octane
4-Ethyl-1-(hex-5-ynyl)-2,6,7-trithiabicyclo[2.2.2]octane
1-(Hex-5-ynyl)-4-phenyl-2,6,7-trioxabicyclo[2.2.2]octane-3-carbonitrile
4-Cyclopropylmethyl-1-(hex-5-ynyl)-2,6,7-trioxabicyclo[2.2.2]octane-3-carbonitrile

By the term "hydrocarbyl" group is meant alkyl, alkenyl (including cyclic alkyl and alkenyl, and alkyl and alkenyl substituted by cyclic alkyl and alkenyl), alkynyl, aryl and aralkyl groups. "Hydrocarbyloxy" means a hydrocarbyl group as defined where linked to oxygen.

By the term "aliphatic" group is meant an alkyl, alkenyl or alkynyl group.

By the term "halo" is meant fluoro, chloro, bromo or iodo.

In a further aspect, the present invention provides a process for the preparation of a compound of the formula (I). The process for the preparation of a compound of the formula (I) may be any method known in the art for preparing analogous compounds, for example:

(i) when Y and $Y^1$ are oxygen and Z is $CH_2O$:

a) by the cyclisation of a compound of the formula (II) :

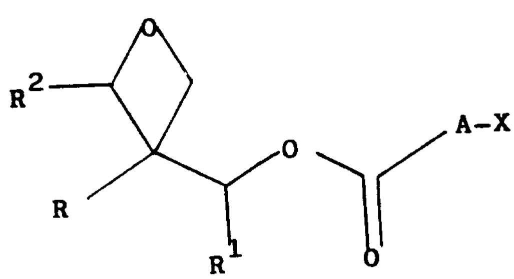

(II)

wherein R to $R^2$, A and X are as hereinbefore defined, in the presence of an acid catalyst. Boron trifluoride etherate is a particularly preferred acid catalyst for this cyclisation which will normally be carried out in an inert solvent, such as a halogenated hydrocarbon, conveniently dichloromethane, at or below ambient temperature, for example between -100 and 50 °C and conveniently between -70 and -25 °C.

The compounds of the formula (II) may be prepared by the reaction of compounds of the formulae (III) and (IV):

(III)

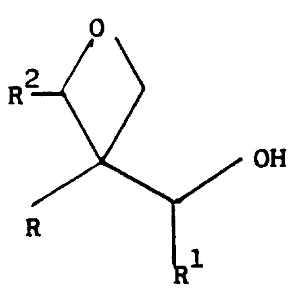

(IV)

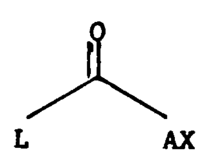

where R to $R^2$, A and X are as hereinbefore defined and L is a leaving group such as halo or hydroxy. This reaction conveniently takes place under conditions well known to those skilled in the art, for example when L is halo in an inert solvent in the presence of base at a non-extreme temperature and when L is hydroxy in an inert solvent in the presence of a condensing agent at a non extreme temperature. When L is halo, halogenated hydrocarbons, such as dichloromethane, are particularly suitable inert solvents and pyridine is a preferred base; when L is hydroxy, dimethylformamide is a suitable solvent, dicyclohexylcarbodiimide is a preferred condensing agent; and the reaction will conveniently be carried out at between -50 and 100 ° C, preferably between 0 and 25 ° C.

The compounds of the formula III may be prepared as described in copending European Patent Applications Nos. 211598 and 216624. The compounds of the formula (IV) may be prepared by methods well known to those skilled in the art, the synthesis of XA-$CO_2$H(XA = 4-ethynylcyclohexyl) is described in Appendix 1.

b) when A contains a terminal C≡C fragment

(bii)when A contains a terminal C}C fragment and X is hydrogen, by the reaction of a strong base with a compound of the formula (VII):

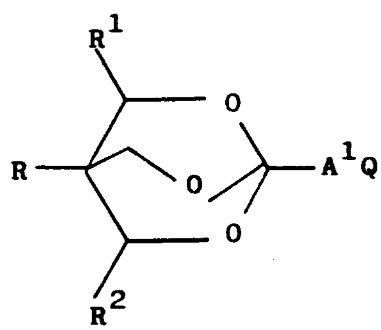

(VII)

wherein R to $R^2$, $A^1$, Y, $Y^1$ and Z are as hereinbefore defined and Q is a group capable of conversion into an ethynyl group, for example a group CH = $C(hal)_2$, (hal)CH = $CH_2$ or -C = $CH_2$ wherein hal is

OPO $(OAlk)_2$

chloro or bromo, Alk is $C_{1-4}$ alkyl.

The reaction is conveniently carried out by methods well known to those skilled in the art, for example when Q is a group -CH = $C(hal)_2$ at about or below room temperature, for example between -70 ° C and 25 ° C, in an inert solvent, conveniently an ether such as tetrahydrofuran.

Butyl lithium is a convenient strong base for use in this reaction. The starting material of the formula (VII) may be prepared by the reaction of a compound of the formula (VIII)

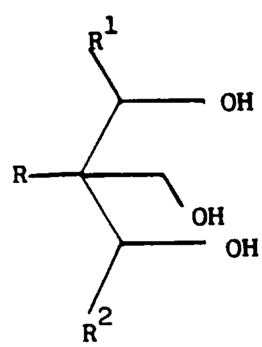

(VIII)

with a compound of the formula $(AlkO)_3$C-$A^1$CH = $C(hal)_2$ or by the cyclisation of a compound analogous to that of the formula (VI) wherein L' is a group Q.

(ii) when n' is 0 $Y^1$ = O or S

Y = O or S

Z = $CH_2$S or $CH_2$O

by the reaction of a compound of the formula (XIV) with a compound of the formula $(AlkO)_3$CAX,

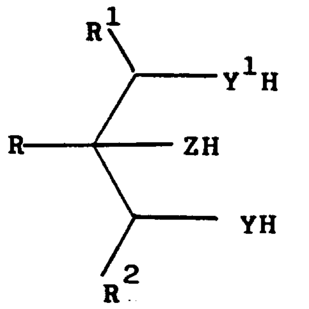

(XIV)

wherein R,$R^1$,$R^2$,A, X, Y, $Y^1$ and Z are as hereinbefore defined and Alk is a $C_{1-4}$ alkyl group. The condensation takes place in the presence of an acid catalyst for example a mineral acid such as concentrated hydrochloric acid or boron trifluoride etherate and/or p-toluenesulphonic. The reaction is conveniently carried out without a solvent, but an inert solvent, conveniently a chlorinated hydrocarbon such as dichloromethane may be added. The reaction can also be carried out in methanol containing hydrogen chloride. The reaction is conveniently carried out at a non-extreme temperature, for example between -70 °C and 150 °C and normally between -10 °C and 150 °C. The compounds of the formula (XIV) may be prepared as described in European Patent Application No 216624, or as described in appendix 2. Compounds of the formula (XIV) wherein $Y = Y^1 = S$, $Z = CH_2S$ and $R^1 = R^2 = H$ may also be prepared by the method described by G. R. Franzen and G. Binsch, J.Amer.Chem.Soc., 1973, 95, 175 and D.J.martin and C.R.Creco, J.Org.Chem., 1968 33, 1275. The compounds of the formula $(AlkO)_3CAX$ may be prepared by a general procedure for the synthesis of orthoesters and is described by S.M.McElvain and R.E.Stam, J.Amer.Chem.Soc., 1955, 77, 4571:

(iii)when Z = $CH_2S$ or $CH_2O$ and Y and $Y^1$ are sulphur by reaction of a compound of the formula (XV)

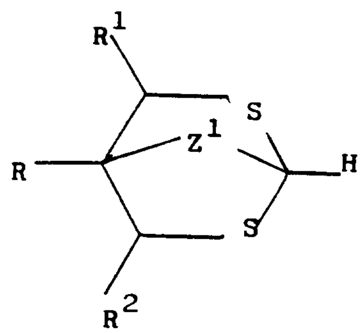

(XV)

with a compound $L^2AX$ wherein R to $R^2$, A and X are as hereinbefore defined, $Z^1$ is $CH_2S$ or $CH_2O$ and $L^2$ is a leaving group eg.halo. The reaction is suitably carried out in the presence of a strong base, such as butyllithium, in an inert solvent, such as an ether and conveniently tetrahydrofuran at a non-extreme temperature, such as between -70 ° and 30 °C. The compound of the formula (XV) can be prepared by the reaction of an analogous compound of the formula (XIV) with $HC(OAlk)_3$ under the conditions described for reaction (ii) above.

(iv) when Y is O and $Y^1$ is O and Z is $CH_2CH_2$ by the reaction of a compound of the formula (XVI) with acid

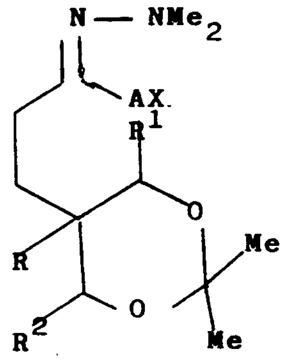

(XVI)

wherein R, $R^1$, $R^2$, A and X are as hereinbefore defined.

The reaction is carried out in acidic conditions, conveniently silica gel, followed by dilute hydrochloric acid at a non-extreme temperature, i.e. between 0° and 100° and conveniently ambient temperature i.e. between 20° and 30°. The compounds of the formula (XVI) may be prepared as illustrated in Appendix 3 herein.

(v) By the interconversion of compounds of the formula (I), for example

a) when it is desired to prepare a compound of the formula (I) wherein A contains a terminal C}C fragment and X is other than hydrogen by the reaction of the corresponding compound wherein X is hydrogen with a compound $X^1$ Hal wherein hal is halogen and $X^1$ is other than hydrogen. This reaction is particularly suitable for the preparation of those compounds wherein X is a $C_{1-4}$ alkyl group or a group $COR^{21}$ wherein $R^{21}$ is a $C_{1-6}$ alkoxy group; or X is a substituted silyl or tin group. The reaction is normally carried out in the presence of a strong base, such as an alkyllithium conveniently butyllithium in an inert solvent, such as an ether, for example tetrahydrofuran, at a non-extreme temperature, for example between -50° and 50°C and conveniently between -10° and 30°C. The starting material, i.e. the unsubstituted alkynylalkyl bicycloalkane may be prepared as described above.

(b) when A contains a terminal C}C fragment and X is hydrogen by desilylation of a compound of the formula (XVII)

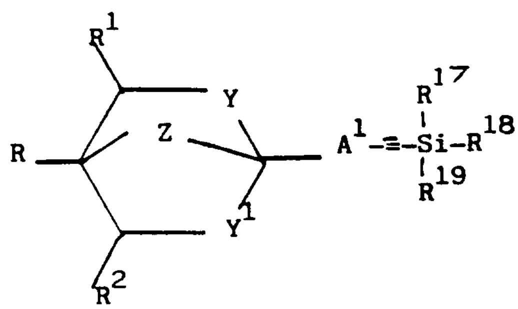

(XVII)

wherein R, $R^1$, $R^2$, $R^{17}$, $R^{18}$, $R^{19}$, Y, $Y^1$, Z and $A^1$ are as defined. This reaction may be carried out by methods well known to those skilled in the art, for example by reaction with tetrabutylammonium fluoride in an ether, such as tetrahydrofuran, at a non-extreme temperature, for example between 0° and 70°C and conveniently at 25°C.

Novel chemical intermediates also form an important aspect of the present invention. Preferred intermediates include those of the formula (II), (V), (VII), (XIV) and (XV).

The compounds of formula (I) may be used to control pests such as arthropods e.g. insect and acarine pests, and helminths, i.e. nematodes. Thus, the present invention provides a method for the control of arthropods and/or helminths which comprises administering to the arthropod and/or helminth or to their environment an arthropodically effective amount of a compound of the formula (I). The present invention also provides a method for the control and/or eradication of arthropod and/or helminth infestations of animals (including humans) and/or of plants, (including trees) and/or stored products which comprises administering to the animal or locus an effective amount of a compound of the formula (I). The present

invention further provides for the compounds of the formula (I) for use in human and veterinary medicine, in public health control and in agriculture for the control of arthropod and/or helminth pests.

The compounds of formula (I) are of particular value in the protection of field, forage, plantation, glasshouse, orchard and vineyard crops, of ornamentals and of plantation and forest trees, for example, cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries.

They are also valuable in the protection of timber (standing, felled, converted, stored or structural) from attack by sawflies (e.g. Urocerus) or beetles (e.g. scolytids, platypodids, lyctids, bostrychids, cerambycids, anobiids).

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

The compounds of general formula I are of particular value in the control of arthropods or helminths which are injurious to, or spread or act as vectors of diseases in man and domestic animals, for example those hereinbefore mentioned, and more especially in the control of ticks, mites, lice, fleas, midges and biting, nuisance and myiasis flies.

The compounds of Formula (I) may be used for such purposes by application of the compounds themselves or in diluted form in known fashion as a dip, spray, fog, lacquer, foam, dust, powder, aqueous suspension, paste, gel, cream, shampoo, grease, combustible solid, vapourising mat, combustible coil, bait, dietary supplement, wettable powder, granule, aerosol, emulsifiable concentrate, oil suspensions, oil solutions, pressure-pack, impregnated article, pour on formulation or other standard formulations well known to those skilled in the art. Dip concentrates are not applied per se, but diluted with water and the animals immersed in a dipping bath containing the dip wash. Sprays may be applied by hand or by means of a spray race or arch. The animal, soil, plant or surface being treated may be saturated with the spray by means of high volume application or superficially coated with the spray by means of light or ultra low volume application. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material, such as that against which animals rub and transfer the material to their skins. Pour-on formulations are dispensed as a unit of liquid of small volume on to the backs of animals such that all or most of the liquid is retained on the animals.

The compounds of Formula (I) may be prepared either as formulations ready for use on the animals, plants or surface or as formulations requiring dilution prior to application, but both types of formulation comprise a compound of Formula (I) in intimate admixture with one or more carriers or diluents. The carriers may be liquid, solid or gaseous or comprise mixtures of such substances, and the compound of Formula (I) may be present in a concentration of from 0.025 to 99% w/v depending upon whether the formulation requires further dilution.

Dusts, powders and granules and other solid formulations comprise the compound of Formula (I) in intimate admixture with a powdered solid inert carrier for example suitable clays, kaolin, bentonite, attapulgite, adsorbent carbon black, talc, mica, chalk, gypsum, tricalcium phosphate, powdered cork, magnesium siliate, vegetable carriers, starch and diatomaceous earths. Such solid formulations are generally prepared by impregnating the solid diluents with solutions of the compound of formula (I) in volatile solvents, evaporating the solvents and, if desired grinding the products so as to obtain powders and, if desired, granulating, compacting or encapsulating the products.

Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 90% w/v of the composition and may be selected from kerosene, ketones, alcohols, xylene, aromatic naphtha, and other solvents known in the formulating art. The concentration of emulsifiers may be varied

within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates. Cationic emulsifiers include benzalkonium chloride and quaternary ammonium ethosuphates.

Amphoteric emulsifiers include carboxymethylated oleic imidazoline and alkyl dimethyl betain.

Vaporising mats normally comprise cotton and cellulose mix compressed into a board of approximately 35 x 22 x 3mm dimensions, treated with up to 0.3ml of concentrate comprising the active ingredient in an organic solvent and optionally an antioxidant, dye and perfume. The insecticide is vaporised using a heat source such as an electrically operated mat heater.

Combustible solids normally comprise of wood powder and binder mixed with the active ingredient and formed into shaped (usually coiled) strips. Dye and fungicide may also be added.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, and other solvents known in the art.

Wettable powders and emulsifiable concentrates will normally contain from 5 to 95% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of Formula (I) in intimate admixture with a dispersing agent and one or more surface active agents.

Aqueous suspensions of a compound of Formula (I) may comprise a suspension in water together with suspending, stabilizing or other agents. The suspensions or solutions may be applied per se or in a diluted form in known fashion.

Greases (or ointments) may be prepared from vegetable oils, synthetic esters of fatty acids or wool fat together with an inert base such as soft paraffin. A compound of Formula (I) is preferably distributed uniformly through the mixture in solution or suspension. Greases may also be made from emulsifiable concentrates by diluting them with an ointment base.

Pastes and shampoos are also semi-solid preparations in which a compound of Formula (I) may be present as an uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of Formula (I) required for treatment.

Aerosol sprays may be prepared as a simple solution of the active ingredient in the aerosol propellant and co-solvent such as halogenated alkanes and the solvents referred to above, respectively. Pour-on formulations may be made as a solution or suspension of a compound of Formula (I) in a liquid medium. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body. Suitably the plastics material is a polyvinyl chloride (PVC).

The concentration of the compound of Formula (I) to be applied to an animal, premises or outdoor areas will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the formulation but in general will lie in the range of from 0.0001% to 0.5% w/w except for undiluted formulations such as pour-on formulations which in general will be deposited at a concentration in the range from 0.1 to 20.0% and preferably 0.1 to 10%. The amount of compound to be applied to stored products in general will lie in the range of from 0.1 to 20ppm. Space sprays may be applied to give an average initial concentration of 0.001 to 1mg of compound of formula (I) per cubic metre of treated space.

The compounds of formula (I) are also of use in the protection and treatment of plant species, in which case an effective insecticidal, acaricidal or nematocidal amount of the active ingredient is applied. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and

1kg/Ha. Typical formulations for agricultural use contain between 0.0001% and 50% of a compound of formula (I) and conveniently between 0.1 and 15% by weight of a compound of the formula (I).

Dusts, greases, pastes and aerosol formulations are usually applied in a random fashion as described above and concentrations of 0.001 to 20% w/v of a compound of Formula (I) in the applied formulation may be used.

The compounds of formula (I) have been found to have activity against the common housefly (Musca domestica). In addition, certain compounds of formula (I) have activity against other arthropod pests including Myzus persicae, Tetranychus urticae, Plutella xylostella, Culex spp. Tribolium castaneum, Sitophilus granarius, Periplaneta amiercana and Blattella germanica. The compounds of formula (I) are thus useful in the control of arthropods e.g. insects and acarines in any environment where these constitute pests, e.g. in agriculture, in animal husbandry, in public health control and in domestic situations.

Insect pests include members of the orders Coleoptera (e.g. Anobium,Ceutorhynchus,Rhynchonhorus, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus or Anthrenus spp.), Lepidoptera (e.g. Ephestia, Mamestra, Earias, Pectinophora, Ostrinia, Trichoplusia, Pieris, Laphygma, Agrotis, Amathes, Wiseana, Tryporysa, Diatraea, Sporganothis, Cydia, Archips, Plutella, Chilo, Heliothis, Spodoptera or Tineola spp.), Diptera (e.g. Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops, Phytomyza, Ceratitis, Liriomyza and Melophagus spp.), Phthiraptera (Malophaga e.g. Damalina spp. and Anoplura e.g. Linoanathus and Haematopinus spp.), Hemiptera (e.g. Aphis, Bemisia,Phorodon, Aeneolamia, Empoasca, Parkinsiella, Pyrilla, Aonidiella, Coccus, Pseudococus, Helopeltis, Lygus, Dysdercus, Oxycarenus, Nezara, Aleurodes, Triatoma, Psylla, Mysus, Megoura, Phylloxera, Adelyes, Niloparvata, Nephrotetix or Cimex spp.), Orthoptera (e.g. Locusta, Gryllus, Schistocerca or Acheta spp.), Dictyoptera (e.g. Blattella, Periplaneta or Blatta spp.), Hymenoptera (e.g. Athalia, Cephus, Atta, Solenopsis or Monomorium spp.), Isoptera (e.g. Odontotermes and Reticulitermes spp.), Siphonaptera (e.g. Ctenocephalides or Pulex spp.), Thysanura (e.g. Lenisma spp.), Dermaptera (e.g. Forficula spp.), Pscoptera (e.g. Peripsocus spp.) and Thysanoptera (e.g. Thrios tabaci),.

Acarine pests include ticks, e.g. members of the genera Boophilus,Ornithodorus, Rhipicephalus, Amblyomma, Hyalomma, Ixodes, Haemaohysalis, Dermacentor and Anocentor, and mites and manges such as Acarus, Tetranychus, Psoroptes, Notoednes, Sarcoptes, Psorergates, Choriptes, Eutrombicula, Demodex, Panonychus, Bryobia, Eriophyes, Blaniulus, Polyphagotarsonemus, Scutigerella, and Oniscus spp.

Nematodes which attack plants and trees of importance to agriculture, forestry, horticulture either directly or by spreading bacterial, viral, mycoplasma or, fungal diseases of the plants, include root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g.R. reniformis); Rotylenchus spp. (e.g. R. robustus); Helicotylenchus spp. (e.g. H. multicinctus);Hemicycliophora spp. (e.g. H. gracilis); Criconemoides spp. (e.g. C. similis); Trichodorus spp. (e.g. T primitivus); dagger nematodes such as Xiphinema spp. (e.g. X. diversicaudatum), Longidorus spp (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

Compounds of the invention may be combined with one or more other pesticidally active ingredients (for example pyrethroids, carbamates and organophosphates) and/or with attractants, repellents, bacteriocides, fungicides, nematocides, anthelmintics and the like. Furthermore, it has been found that the activity of the compounds of the invention may be enhanced by the addition of a synergist or potentiator, for example: one of the oxidase inhibitor class of synergists, such as piperonyl butoxide or propyl 2-propynylphenylphosphonate; a second compound of the invention; or a pyrethroid pesticidal compound. When an oxidase inhibitor synergist is present in a formula of the invention, the ratio of synergist to compound of Formula (I) will be in the range 25:1-1:25 eg about 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin) and organic or inorganic bases e.g. trialkylamines such as triethylamine which can act as basic stabilises and as scavengers.

The following Examples illustrate preferred aspects of the invention. All temperatures are in degrees Celsius.

Example I

1-(Hex-5-ynyl)-4-oropyl-2,6,7-trioxabicyclo[2.2.2]octane.

(i) A solution of methanesulphonyl chloride (23.7ml) in dry dichloromethane (25ml.) was added dropwise to a solution of hex-5-yn-1-ol (Lancaster Synthesis, 25g) and triethylamine (47.3ml) in dichloromethane (300ml), stirred under a nitrogen atmosphere at -70°. The resulting mixture was allowed to warm to room temperature over 16 hours. The mixture was then washed with water, dilute hydrochloric acid, saturated sodium bicarbonate solution and brine before drying over anhydrous magnesium sulphate and evaporation in vacuo. Hex-5-ynyl methanesulphonate was obtained as an oil (43.6g) and was used without purification.

Nuclear magnetic resonance spectrum (NMR) was as follows: $^1H$ (p.p.m from TMS in $CDCl_3$, integral, number of peaks, $J_{Hz}$): 4.15, 2H, t, $J_{Hz}$ 6; 3.0, 3H, s; 2.4-1.4, 7H, m.

(ii) A mixture of hex-5-ynyl methanesulphonate (43.6g) and potassium cyanide (24g) in 20% aqueous ethanol (150ml) was heated under reflux for 4 hours and then stirred at room temperature overnight. Water (600ml) was added and the mixture was extracted with diethyl ether. The organic extracts were washed with water and brine before drying over anhydrous magnesium sulphate and evaporation in vacuo.

Distillation gave 6-cyano-1-hexyne (20.1g) as a colourless oil (b.p. 82-95°, 15mm Hg).

Infrared spectrum (IR) (liquid film): 3340, 2990, 2920, 2300, 2160, 1485, 1450, 1355, 670cm$^{-1}$.

(iii) A mixture of 6-cyano-1-hexyne and 10% aqueous potassium hydroxide solution was heated under reflux for 6 hours. The resulting solution was extracted with cloroform. The aqueous phase was acidified to pH 1 with concentrated hydrochloric acid and then reextracted with dichloromethane. The dichloromethane extracts were washed with brine, dried over anhydrous magnesium sulphate and then evaporat evaporated in vacuo.

Distillation gave hept 6-ynoic acid.

Infrared spectrum (IR) (liquid film): 3340, 2980, 2160, 1730, 1435, 1310, 1255, 955, 660 cm$^{-1}$.

(iv) A mixture of hept-6-ynoic acid and thionyl chloride in benzene was heated under reflux for 3 hours. The resulting solution was cooled and then evaporated in vacuo. The acid halide thus obtained was taken up in ether (5ml) and added, dropwise, to a stirred solution of 3-hydroxymethyl-3-n-propyloxetane (1.2g) and pyridine (0.8ml) in dry ether (20ml.). The reaction mixture was stirred at room temperature for 16 hours. After this time the organic phase was washed with water, 5% hydrochloric acid, saturated sodium bicarbonate solution and brine before drying over anhydrous magnesium sulphate and then evaporation in vacuo. The residue was purified by chromatography on silica, pre-eluted with hexane containing 1% triethylamine. Elution with hexane/ether mixtures gave (3-propyloxetan-3-yl)methyl hept-6-ynoate.

(v) Boron trifluoride etherate (0.18ml) was added to a stirred solution of (3-propyloxetan-3-yl)methyl hept-6-ynoate in dry dichloromethane at -70°. The mixture was allowed to warm to room temperature over 16 hours. Triethylamine was then added and the solvent was removed in vacuo. The residue was partitioned between diethyl ether and water. The organic phase was separated and further washed with water and brine before drying over anhydrous magnesium sulphate. The solvent was evaporated in vacuo and the residue was purified by column chromatography on alumina eluting with 1 : 6 dichloromethane : hexane saturated with ammonia. 1-(Hex-5-ynyl)-4-propyl-2,6,7-trioxabicyclo[2.2.2]octane was obtained as a colourless oil which crystallised on trituration with hexane.

Gas-liquid chromatography (g.l.c): OV-17 at 175° produced one peak.

Example II

1-(hex-5-ynyl)-4-propyl-3-trifluoromethyl-2,6,7-trioxabicyclo[2.2.2]octane

Using the method described in stages (iv) and (v) of Example I and starting from hept-6-ynoic acid and 3-(1-hydroxy-2,2,2-trifluoroethyl)-3-n-propyloxetane:
1-(Hex-5-ynyl)-4-propyl-3-trifluoromethyl-2,6,7-trioxabicyclo[2.2.2] octane was obtained.

Example III

1-(hex-5-ynyl)-4-propyl-2,6,7-trioxabicyclo[2.2.2]octane-3-carbonitrile

(i) A solution of dimethyl sulphoxide (12ml) in dry dichloromethane (4.0ml) was added to a solution of oxalyl chloride (7.4ml) in dichloromethane (25ml) stirred at -70° under nitrogen. After the addition was complete the resulting mixture was stirred for a further 5 minutes at -70° before a solution of 3-hydroxymethyl-3-n-propyl oxetane (10.0g) in dichloromethane (25ml) was added, dropwise, over 10 minutes. The resulting mixture was allowed to stir for a further 30 minutes when neat triethylamine (54ml) was added over approximately 30 minutes. The reaction mixture was allowed to warm to room temperature over 3 hours when it was poured into water. The organic phase was separated and the aqueous layer was further extracted with dichloromethane. The combined organic extracts were washed with dilute hydrochloric acid, saturated sodium bicarbonate and brine. The resulting organic phase was dried over anhydrous magnesium sulphate and evaporated in vacuo to give 3-formyl-3-n-propyloxetane (10.5g) (European Patent Application No. 216624) as a yellow oil.

(ii) A mixture of hept-6-ynoic acid and thionyl chloride (1.95ml) in benzene (25 ml) was heated at reflux for 3 hours. The resulting solution was allowed to cool and then evaporated in vacuo. The acid chloride thus obtained was added to a stirred solution of 3-formyl-3-n-propyloxetane (1.14g) in ether (50ml) followed by a solution of sodium cyanide (0.61g) in water (1ml). The resulting mixture was stirred briskly at room temperature for 16 hours. After this time the reaction mixture was washed with water, saturated sodium bicarbonate and brine, before drying over anhydrous magnesium sulphate. The solvent was removed in vacuo and the residue was purified by column chromatography on silica, pre-eluted with hexane containing 1% triethylamine. Gradient elution with hexane/ether mixtures gave 1-cyano-1-(3-propyloxetan-3-yl) methyl hept-6-ynoate as a colourless oil (1.2g).

(iii)Boron trifluoride etherate (0.25ml) was added to a solution of 1-cyano-1-(3-propyloxetan-3-yl)methyl hept-6-ynoate in dry dichloromethane (10ml) stirred at -70° under a nitrogen atmosphere. The resulting solution was allowed to warm to room temperature overnight. Triethylamine (0.38ml.) was added and the solvent was removed under vacuum. The residue was partitioned between water and diethyl ether. The organic phase was separated and washed with water and brine before drying over anhydrous magnesium sulphate and evaporation in vacuo. The residue was purified by column chromatography on alumina eluting with 1:4 dichloromethane:hexane saturated with ammonia. 1-(hex-5-ynyl)-4-propyl-2,6,7-trioxabicyclo [2.2.2]octane-3-carbonitrile was obtained.

In an analogous manner the following compounds were prepared :

4-n-Butyl-1(hex-5-ynyl)-2,6,7-trioxabicyclo[2.2.2]octane-3-carbonitrile

4-t-Butyl-1-(hex-5-ynyl)-2,6,7-trioxabicyclo[2.2.2]octane-3-carbonitrile

(Preparation of 2-t-butyl-2-hydroxymethylpropan-1,3-diol, Y. Ozoe and M.Eto, Agric. Biol.Chem. 1982, 46, 411).

4-Cyclopropylmethyl-1-(hex-5-ynyl)-2,6,7-trioxabicyclo[2.2.2]octane-3carbonitrile, 1-(hex-5-ynyl)-4-phenyl-2,6,7-trioxabicyclo[2.2.2]- octane-3-carbonitrile, 1-(hex-5-ynyl)-4-i-butyl-2,6,7-trioxabicyclo[2.2.2]octane-3-carbonitrile and 1-(hex-5-ynyl)-4-(2-methylprop-2- enyl)- 2,6,7-trioxabicyclo[2.2.2]octane-3-carbonitrile were prepared from 3-cyclopropylmethyl-3-formyloxetane, 3-formyl-3-phenyloxetane, 3-formyl-3-i-butyloxetane and 3-formyl-3-(2-methylprop-2-enyl)oxetane respectively. 3-Cyclopropylmethyl-3-formyloxetane, 3-formyl-3- phenyloxetane, 3-formyl-3-i-butyloxetane and 3-formyl-3-(2-methylprop-2-enyl)oxetane were prepared from diethyl cyclopropylmethylmalonate (J.A.Arvin and R. Adams, J. Amer. Chem.Soc. 1928, 50, 1985), diethyl phenylmalonate (Aldrich), diethyl i-butylmalonate (Beilstein. 2, 683) and diethyl 2-methylprop-2-enylmalonate (W.J.Doran and H.A. Shonle, J. Amer. Chem. Soc. 1937, 59, 1625).

Example IV

4-t-Butyl-1-(hex-5-ynyl)-2,6,7-trioxabicyclo[2.2.2]octane

Method 1

Using the procedure described in stages (iv) and (v) of Example 1 and starting from hept-6-ynoic acid and 3-t-butyl-3-hydroxymethyloxetane (2-t-butyl-2-hydroxymethylpropan-1,3-diol was prepared by the method of Y.Ozoe and M.Eto Agric.Biol. Chem. 1982, 46, 411), 4-t-butyl-1-(hex-5-ynyl)-2,6,7-trioxa- bicyclo-

[2.2.2]octane was prepared.

Gas-liquid chromatography (g.l.c.): OV-17 at 230° produced one peak.

Method 2

A mixture of 2-t-butyl-2-hydroxymethylpropan-1,3-diol (0.75g) and trimethyl orthohept-6-ynoate (0.75g) (see Example XI) was heated at 50° until homogeneous. One drop of concentrated hydrochloric acid was added and the mixture was heated at 135°, under a current of nitrogen for 10 minutes. The mixture was cooled and chromatographed on alumina, eluting with 1:6 dichloromethane: hexane saturated with ammonia.

4-t-Butyl-1-(hex-5-ynyl)-2,6,7-trioxabicyclo[2.2.2]octane was obtained as colourless crystals (0.38g, recrystallised from hexane).

In an analogous manner 1-(hex-5-ynyl)-4-phenyl-2,6,7-trioxabicyclo[2.2.2]- octane was prepared from 2-hydroxymethyl-2-phenylpropan-1,3-diol (prepared from diethyl 2-phenylmalonate through diethyl 2-benzyloxymethyl 2-phenylmalonate and 2-benzyloxymethyl 2-phenyl propane-1,3-diol.

Example V

4-t-Butyl-1-(6-trimethylsilylhex-5-ynyl)-2,6,7-trioxabicyclo [2.2.2]octane

n-Butyllithium (0.31ml. of 1.6M solution, in hexane) was added to a stirred solution of 4-t-butyl-1-(hex-5-ynyl)-2,6,7-trioxabicyclo[2.2.2]octane (100mg) in dry tetrahydrofuran (4.0ml), at 0°, under nitrogen. The reaction mixture was stirred at 0° for 15 minutes. Trimethylsilyl chloride (63 l) was added and the mixture was allowed to warm up to 20° over a period of 2 hours. Water (0.5ml) was added and the solvent was removed in vacuo. Water was added and the aqueous mixture was extracted with diethyl ether. The ethereal extracts were washed with brine and dried over anhydrous magnesium sulphate. The solvent was removed in vacuo. The residue was purified by chromatography on alumina (Alumina Woelm TSC) eluting with 1:6 dichloromethane:hexane saturated with ammonia.

4-t-Butyl-1-(6-trimethylsilylhex-5-ynyl)-2,6,7-trioxabicyclo[2.2.2]octane was obtained as colourless crystals (60mg) (m.p. 87-90.5°). Gas-liquid chromatography (g.l.c.): OV-17 at 230° produced one peak.

Example VI

1-(Hex-5-ynyl)-4-n-propyl-2-oxa-6,7-dithiabicyclo[2.2.2]octane

(i) A stirred solution of 6-cyanohex-1-yne (4.0 g) (Synthesis: see example II, stage ii) in dry methanol (30ml) and dry diethyl ether (30 ml) was saturated with hydrogen chloride gas and the temperature was maintained between -10° and 0°. The solution was diluted with dry diethyl ether (120ml) and left at -20° for 24 hours. The white crystalline solid was filtered off and dried in vacuo to give methyl iminohept-6-ynoate hydrochloride.

(ii) Dry methanol (33ml) was added to methyl iminohept-6-ynoate hydrochloride (38.4g), under a current of dry nitrogen. Hexane (750ml) was added and the mixture was stirred at 20° for 6 hours. The mixture was allowed to stand overnight and the supernatant hexane solution was removed by decantation and evaporated in vacuo to give trimethyl orthohept-6-ynoate, a colourless oil (25.0g).

(iii)Using methodology described in European Patent 216625, and starting from trimethyl orthohept-6-ynoate and 2-hydroxymethyl-2-n-propyl- propan-1,3-dithiol (synthesis also described in European Patent 216625), 1-(hex-5-ynyl)-4-n-propyl-2-oxa-6,7-dithiabicyclo[2.2.2] octane was prepared.

2-Hydroxymethyl-2-n-propylpropan-1,3-dithiol was also prepared as follows:-

(i) 3-n-Propyloxetan-3-ylmethyl methanesulphonate was prepared from 3-hydroxymethyl-3-n-propyloxetane and methanesulphonyl chloride using methodology outlined in stage i) of Example II.

(ii) A solution of benzyl mercaptan (25.0ml) in dry dimethylformamide (100ml) was stirred at 0°C, under a current of nitrogen. Sodium hydride (6.0g., 80% dispersion in oil) was added carefully and the mixture was stirred at 0° for 1 hour. 3-n-Propyloxetan-3-ylmethyl methanesulphonate (10.0g.) was added and the mixture was stirred at 0°C for 1 hour. The mixture was refluxed with stirring for 6 hours. The mixture was cooled and poured into water. The aqueous mixture was extracted with diethyl ether. The ethereal extracts were washed with water, dried over anhydrous magnesium sulphate and evaporated in vacuo. The residue was chromatographed on silica, eluting with 1:4; diethyl ether: hexane. 2,2-Di-(benzylthiomethyl)pentan-1-ol was obtained as a pale yellow oil (15.6g.)

Mass spectrum (chemical ionisation):

M + 1 361

(iii)2,2-Di-(benzylthiomethyl)pentan-1-ol (8.0g.) in dry diethyl ether (150ml) was added to liquid ammonia (500ml) which was stirred under nitrogen at -70°. Sodium (8.0g.) was added in small pieces and the mixture was stirred at -70° for 3 hours. The mixture was allowed to warm to 20° and solid ammonium chloride (20g.) was added. This was followed by careful addition of methanol (100ml) to destroy excess sodium. Water (200ml) was added and the aqueous mixture was extracted with diethyl ether. The ethereal extracts were dried over anhydrous magnesium sulphate and evaporated in vacuo. 2-Hydroxymethyl-2-n-propylpropan-1,3-dithiol was obtained as a colourless oil (4.6g.).

In an analogous manner 4-n-butyl-1-(hex-5-ynyl)-2-oxa-6,7-dithiabicyclo[2.2.2]octane, 4-t-butyl-1-(hex-5-ynyl)-2-oxa-6,7-dithiabicyclo [2.2.2]octane, 4-i-butyl-1-(hex-5-ynyl)-2-oxa-6,7-dithiabicyclo[2.2.2] octane, 1-(hex-5-ynyl)-4-phenyl-2-oxa-6,7-dithiabicyclo[2.2.2]octane and 4-cyclopropylmethyl-1-(hex-5-ynyl)-2-oxa-6,7-dithiabicyclo[2.2.2] octane were prepared from trimethyl orthohept-6-ynoate and 2-n-butyl-2-hydroxymethylpropan-1,3-dithiol 2-t-butyl-2-hydroxymethylpropan-1,3-dithiol 2-i-butyl-2-hydroxymethylpropan-1,3-dithiol, 2-hydroxymethyl-2-phenylpropan1,3-dithiol and 2-cyclopropylmethyl-2-hydroxy-methylpropan-1,3-dithiol respectively.

Example VII

1-Hex-5-ynyl-4-n-propyl-2,6,7-trithiabicyclo[2.2.2]octane

Method 1

Boron trifluoride etherate (0.20ml) was added to a stirred solution of 2-mercaptomethyl-2-n-propyl-propan-1,3-dithiol (0.30g) (Synthesis described in Example XXXI) and trimethyl orthohept-6-ynoate (0.30g) in dry dichloro- methane (10ml) at 20°, under nitrogen. The mixture was stirred at 20° for 5 hours and triethylamine (1.0ml) was added. Water was added and the aqueous mixture was extracted with diethyl ether. The ethereal extracts were dried over anhydrous magnesium sulphate and the solvent was removed in vacuo. The residue was chromatographed on alumina, eluting with 1:10 dichloromethane: hexane saturated with ammonia. The volatile impurity (methyl hept-6-ynoate) was removed in vacuo (130°, 10 mm Hg).

Recrystallisation of the residue from hexane gave 1-(hex-5-ynyl)-4-n-propyl-2,6,7-trithiabicyclo[2.2.2]-octane as a colourless solid (10mg).

Method 2

A solution of trimethyl orthohept-6-ynoate (6.0g.) in dry methanol (24ml.) was stirred at 0°C, under nitrogen. 2-Mercaptomethyl-2-n-propylpropan-1,3-dithiol (3.0g) in dry methanol (10ml) was added and this was followed by methanol, saturated with hydrogen chloride (1.0ml). After 20 minutes stirring, at 0°C, dry triethylamine (3.0ml) was added. Water (100 ml) was then added and the mixture was extracted with diethyl ether. The ethereal extracts were dried over anhydrous magnesium sulphate and evaporated in vacuo. The residue was chromatographed on alumina, eluting with 1:10; dichloromethane : hexane saturated with ammonia. The volatile components were removed in vacuo (kugelrohr at 130°, 0.5 mm Hg). The residue was crystallised from hexane.

1-(Hex-5-ynyl)-4-n-propyl-2,6,7-trithiabicyclo[2.2.2]octane was obtained as colourless crystals (0.90g.).

Using analogous methodology (Method 2) 4-ethyl-1-(hex-5-ynyl)-2,6,7-trithiabicyclo[2.2.2]octane and 4-i-but-yl-1-(hex-5-ynyl)-2,6,7-trithiabicyclo [2.2.2]octane were prepared from trimethylorthohept-6-ynoate and 2-ethyl-2-mercaptomethylpropan-1,3-dithiol and 2-i-butyl-2-mercaptomethylpropan-1,3-dithiol respectively.

Example VIII

1-(Hex-5-ynyl)-3-methyl-4-n-propyl-2-oxa-6,7-dithiabicyclo [2.2.2]octane

i) A solution of 2,2-di-(benzylthiomethyl)pentan-1-ol (1.0g Example XI) in dry dichloromethane (10 ml) was added to a stirred suspension of pyridinium chlorochromate (1.8g) and anhydrous sodium acetate (0.11g) in dry dichloromethane (25 ml) at 0°C, under a current of nitrogen. The reaction mixture was allowed to warm to 20°C and then stirred for 2 hours. Dry diethyl ether was added and the mixture stirred for 30 minutes. The ethereal extracts were decanted off and the residue was washed with further portions of diethyl ether. The combined ethereal extracts were dried over anhydrous magnesium

sulphate and the solvent was removed in vacuo. The residue was purified by chromatography on a mixture of silica and charcoal, eluting with diethyl ether. 2,2-Di-(benzylthiomethyl)pentanal was obtained as a pale yellow oil (0.27g).

Mass spectrum (Chemical Ionisation)

M + 1 359

ii) A solution of 2,2-di-(benzylthiomethyl)pentanal (3.54g) in dry diethyl ether was added dropwise to a stirred solution of methyl magnesium iodide [prepared from methyl iodide (1.2ml) and magnesium (0.48g)] in dry diethyl ether (60 ml). The reaction mixture was refluxed for 2 hours and then cooled. Saturated aqueous ammonium chloride solution was added. The mixture was stirred for 30 minutes and extracted with diethyl ether. The ethereal extracts were dried over anhydrous magnesium sulphate and the solvent was removed in vacuo. 3,3-Di-(benzylthiomethyl)hexan-2-ol was obtained as a yellow oil (1.7g)

Mass Spectrum (Chemical Ionisation)

M + 1 375

1-(Hex-5-ynyl)-3-methyl-4-n-propyl-2-oxa-6,7-dithiabicyclo[2.2.2]octane was prepared from 3,3-di-(benzylthiomethyl)hexan-2-ol using methodology described in Example VI.

Example IX

1-(Hex-5-ynyl)-4-n-propyl-2,6-dioxabicyclo[2.2.2]octane

(i) A solution of trimethylsilylacetylene (12.0g., Aldrich) in dry tetrahydrofuran (100ml.) was stirred at 0°, under a current of nitrogen and n-butyllithium (76.5ml. of 1.6M solution in hexane) was added dropwise. The solution was stirred for 30 minutes and a solution of 1-chloro-3-iodopropane (25.0g.) in dry tetrahydrofuran (75ml.) was added. The reaction mixture was allowed to warm to 20° and stirred for 18 hours. The mixture was poured into water and the aqueous mixture was extracted with diethyl ether. The ethereal extracts were washed with water and dried over anhydrous magnesium sulphate. The solvent was removed in vacuo and the residue distilled. 5-Chloro-1-trimethylsilylpent-1-yne was obtained as a colourless oil (12.7g., b.p. 67-72°, 15mm. Hg.).

ii) A mixture of 5-chloro-1-trimethylsilylpent-1-yne (13.5g) and sodium iodide (29g) in butanone (100ml) was heated at reflux for 10 hours. After this time the solvent was removed in vacuo and the residue partitioned between diethyl ether and water. The organic phase was separated, washed with water and brine before drying over anhydrous magnesium sulphate. The solvent was removed under reduced pressure to leave 5-iodo-1-trimethylsilylpent-1-yne as a colourless oil(19.3g).

(iii)n-Butyllithium (19.0ml., 1.6M solution in hexane) was added dropwise to a stirred solution of acetone N,N-dimethylhydrazone (2.6g.) (ref. R.H.Wiley et al J.Oro.Chem. 1957, 22, 204) in dry tetrahydrofuran (40ml.) at -70°, under a current of nitrogen. The resulting solution was stirred for 30 minutes and a solution of 5-iodo-1-trimethylsilylpent-1-yne (8.1g.) in dry tetrahydrofuran (30ml.) was added dropwise and the reaction mixture was stirred at -70° for 1 hour, allowed to warm to 0° and stirred for 2 hours. The reaction mixture was cooled to -70° and a second portion of n-butyllithium (19ml. 1.6M solution in hexane) was added dropwise. The mixture was stirred at -70° for 15 minutes, allowed to warm to 0° and stirred for 30 minutes. A solution of 5-iodomethyl-2,2-dimethyl-5-n-propyl-1,3-dioxane (9.0g. European Patent 216625) in dry tetrahydrofuran (30ml.) was added. The mixture was stirred at 0° for 30 minutes and 48 hours at 20°. The solvent was removed in vacuo and the residue was poured into water. The aqueous mixture was extracted with diethyl ether and the ethereal extracts were washed with water, dried over anhydrous magnesium sulphate and evaporated in vacuo. Chromatography of the residue on silica, eluting with diethyl ether : hexane 1:9 gave a yellow oil which consisted of 2,2-dimethyl-5-(3-oxo-9-trimethylsilylnon-8-ynyl)-5-n-propyl-1,3-dioxane and 7-oxo-1-trime- thylsilyloct-1-yne in the ratio of 3:1 (1.4g.). The above mixture in tetrahydrofuran (25ml.) and hydrochloric acid (50ml.,1N solution) was stirred vigorously at 20° for 1 hour. The tetrahydro- furan was removed in vacuo and the residue was extracted into diethyl ether. The ethereal extracts were dried over magnesium sulphate and the solvent was removed in vacuo. 4-n-Propyl-1-(6-trimethylsilylhex-5-ynyl) 2,6-dioxabicyclo[2.2.2]octane was obtained as a colourless oil (1.06g.) and was used without further purification.

Mass spectrum (Chemical Ionisation)

M + 1 309

(iv) Tetrabutylammonium fluoride solution (1.0M.,solution 4.0ml.) was added to a stirred solution of 4-n-propyl-1-(6-trimethylsilylhex-5-ynyl)-2,6-dioxabicyclo[2.2.2]octane in dry tetrahydrofuran (40ml.) at 20° and the mixture was stirred for 1 hour. The solvent was removed in vacuo and the residue was extracted

with diethyl ether and water. The ethereal extracts were dried over anhydrous magnesium sulphate and evaporated in vacuo. The residue was chromatographed on alumina eluting with dichloromethane : hexane 1:9, saturated with ammonia. 1-(Hex-5-ynyl)-4-n-propyl-2,6-dioxabicyclo[2.2.2]octane was obtained as a colourless oil (0.45g.)

## Example X

### 4-Ethoxymethyl-1-(hex-5-ynyl)-2,6,7-trioxabicyclo[2.2.2]octane -3-carbonitrile

i) To a stirred solution of 5,5-di-(hydroxymethyl)-2,2-dimethyl-1,3-dioxane (5.0g) (Beilstein 19, II, 93) in dry dimethylformamide (50ml) under nitrogen, at 20°, was added sodium hydride (0.68g, 80% dispersion in oil). The mixture was stirred at 80° for 2 hours and cooled. Ethyl iodide (4.4g) in dry dimethylformamide (40ml) was added and the mixture was heated at 110° for 3 hours. The mixture was cooled and poured into water. The aqueous mixture was extracted with diethyl ether. The ethereal extracts were washed with brine, dried over anhydrous magnesium sulphate and evaporated in vacuo. 2,2-Dimethyl-5-ethoxymethyl-5-hydroxymethyl-1,3-dioxane was obtained as a pale yellow oil (1.2g) and was used without further purification.

ii) A mixture consisting of 2,2-dimethyl-5-ethoxymethyl-5-hydroxymethyl-1,3-dioxane (15.0g) and Amberlyst "15" (3.0g) in methanol (500ml) containing water (10ml) was refluxed with stirring for 4 hours. The mixture was filtered and the filtrate was evaporated in vacuo. 2-Ethoxymethyl-2-hydroxymethylpropan-1,3-diol was obtained as a viscous oil (10.5g) and was used without further purification.

iii) 4-Ethoxymethyl-1-(hex-5-ynyl)-2,6,7-trioxabicyclo[2.2.2]octane-3-carbonitrile was prepared from 2-ethoxymethyl-2-hydroxymethylpropan-1,3-diol using methodology described in Example II.

In an analogous manner 1-(hex-5-ynyl)-4-methoxymethyl-2,6,7-trioxabicyclo[2.2.2]octane-3-carbonitrile was prepared.

## Example XI

### 4-t-Butyl-1-(hex-5-ynyl)-2,6,dioxa-7-thiabicyclo[2.2.2]octane

4-t-Butyl-1-(hex-5-ynyl)-2,6,dioxa-7-thiabicyclo[2.2.2]octane was prepared from trimethyl orthohept-6-ynoate and 2,2-di-(hydroxymethyl)-3,3-dimethyl- butan-1-thiol (European Patent 216625) using methodology described in European Patent 216625.

## Example XII

### 4-(2,2-Dichlorocyclopropymethyl)-1-(hex-5-ynyl)-2,6,7-trioxabicyclo[2.2.2]octane

i) 2-Hydroxymethyl-2-(prop-2-enyl)propan-1,3-diol triacetate (2.2g) (Example XVII) was heated at 130°, with stirring. Sodium trichloroacetate (5.0g) was added over 2 hours and the reaction mixture was heated at 155° for 24 hours. The mixture was cooled and partitioned between diethyl ether and water. The ethereal extracts were dried over anhydrous magnesium sulphate and evaporated in vacuo. The residue was purified by chromatography on silica, eluting with diethyl ether : hexane, 1 : 4.

2-(2,2-Dichlorocyclopropylmethyl)-2-hydroxymethylpropan-1,3-diol triacetate was obtained as a colourless oil (1.4g).

Mass Spectrum (Chemical Ionisation):

Ammonia as Ionising Gas. M + 18 372.

ii) Using methodology described in Example I, stage (i), 2-(2,2-dichloro-cyclopropylmethyl)-2-hydroxymethylpropan-1,3-diol was prepared from 2-(2,2-dichlorocyclopropylmethyl)-2-hydroxymethylpropan-1,3-diol tri-acetate.

iii) Using methodology described in Example IV, Method 2, 4-(2,2-dichloro-cyclopropylmethyl)-1-(hex-5-ynyl)-2,6,7-trioxabicyclo[2.2. 2]octane was prepared from 2-(2,2-dichlorocyclopropylmethyl)-2-hydroxymethylpropan-1,3-diol and trimethylorthohept-6-yn-oate.

Formulations

1. Emulsifiable Concentrate

| | |
|---|---|
| Compound of formula (I) | 10.00 |
| Ethylan KEO | 20.00 |
| Xylene | 67.50 |
| Butylated Hydroxyanisole | 2.50 |
| | 100.00 |

2. Wettable Powder

| | |
|---|---|
| Compound of formula (I) | 25.00 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

3. Dust

| | |
|---|---|
| Compound of formula (I) | 0.50 |
| Butylated Hydroxyanisole | 0.10 |
| Talc | 99.40 |
| | 100.00 |

4. Bait

| | |
|---|---|
| Compound of formula (I) | 40.25 |
| Icing Sugar | 59.65 |
| Butylated hydroxy toluene | 0.10 |
| | 100.00 |

5. Lacquer

| | |
|---|---|
| Compound of formula (I) | 0.1 |
| Piperonyl Butoxide | 0.5 |
| Butylated Hydroxyanisole | 10.1 |
| High aromatic white spirit | 92.0 |
| | 100.00 |

6. Aerosol

| | |
|---|---|
| Compound of formula (I) | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

7. Spray

| | |
|---|---|
| Compound of formula (I) | 0.1 |
| Butylated Hydroxyanisole | 0.1 |
| Xylene | 10.0 |
| Odourless Kerosene | 89.8 |
| | 100.00 |

8. Potentiated Spray

| | |
|---|---|
| Compound of formula (I) | 0.1 |
| Piperonyl Butoxide | 0.5 |
| Butylated Hydroxyanisole | 0.1 |
| Xylene | 10.1 |
| Odourless Kerosene | 89.2 |
| | 100.00 |

BIOLOGICAL ACTIVITIES

The following examples illustrate, in a non-limiting manner, the pesticidal activity of compounds of formula (I).

Spray Tests

The activity of the compounds of the invention were tested by dissolving the compounds in acetone (5%) and then diluting in water: 'Synperonic' (94.5%: 0.5%) to give a water emulsion. The solution was then used to treat the following insects.

Musca domestica

20 female Musca were contained in a cardboard cylinder with gauze over both ends. Solution containing the compound was sprayed onto the insects so enclosed and mortality assessed after 48 hours at 25°.

The following compounds were active at <1000p.p.m.
4, 45, 50, 56, 71, 72, 76, 84.

The following compounds were active at <200p.p.m.
6, 9, 15, 16, 17, 29, 30, 31, 33, 46, 47, 48, 57, 58, 78, 79.

Sitophilus granarius and Tribolium castaneum

20 adult Sitophilus and Tribolium were added to 10g wheat which had been previously treated with 2ml of the solution containing the compounds. Mortality was assessed after 6 days at 25°C.

The following compounds were active against Sitophilus granarius at <1000 p.p.m.:
19, 50, 56, 84.

The following compounds were active against Sitophilus granarius at <200 p.p.m.:-
4, 6, 9, 15, 16, 17, 29, 30, 31, 33, 45, 47, 48, 57, 58, 72, 76, 78, 79.

The following compounds were active against Tribolium castaneum at <1000 p.p.m:-
4, 6, 17, 33, 57, 58, 71, 76, 79.

The following compounds were active against Tribolium castaneum at <200 p.p.m:-
9, 15, 16, 46.

Myzus persicae

10 adult Myzus were placed on a leaf disc of chinese cabbage. 24 hours later the disc was sprayed with a solution containing the compound. Mortality was assessed after 2 days at 25°.

The following compounds were active at <1000 p.p.m.:-
4, 9, 17, 46, 56, 71, 78, 79.
The following compounds were active at <200 p.p.m.:-
6, 45, 47, 48, 57, 58, 76.

Plutella xylostella

7 Plutella larvae were sprayed with the solution containing the compound and added to a chinese cabbage leaf which had been similarly sprayed and left to dry. Alternatively 8-10 Plutella larvae were put onto leaf discs and sprayed with the solution containing the compound. Mortality was assessed after 2 days at 25°.
The following compounds were active at <1000 p.p.m.:-
15, 16, 29, 31, 33, 48, 55, 56, 58, 71, 78, 79.
The following compounds were active at <200 p.p.m.:-
9, 46, 47, 57.

Tetranychus urticae

Leaf discs containing mixed population of Tetranychus urticae were sprayed with the solution of the compound. Mortality was assessed after 2 days at 25°C.
The following compounds were active at <1000 p.p.m.:-
78.

Additional spray tests

The activities of the compounds were investigated further. The compounds were dissolved in acetone (75%) and water (25%) was added. The solution was then used to spray the following insects:-

Aphis fabae

A mixed population of Aphis fabae was tested on Nasturtium leaf.
The following compounds were active at <1000 p.p.m.:-
4, 6, 16, 17, 29, 30, 31, 33, 46, 47, 50.

Macrosteles fascifrons

Adult Macrosteles fascifrons were tested on wheat seedlings.
The following compounds were active at <1000 p.p.m.:-
6, 9, 16, 17, 29, 31, 33, 46, 47.

Tetranychus urticae

A mixed population of Tetranychus urticae was tested on bean leaves. The following compounds were active at <1000 p.p.m.:-
9, 16, 30, 31, 33, 46, 47, 50.

Diabrotica undecimpunctata

3rd Instar Diabrotica undecimpunctata were tested on filter paper.
The following compounds were active at <1000 p.p.m.:-
4, 6, 16, 17, 29, 30, 31, 33, 46, 47.

Topical Application Tests

The activity of compounds of the invention against unanaesthatised female Musca domestica (WRL strain) was demonstrated by the topical application to the test insect of a solution of the compound under test with piperonyl butoxide in butanone. Mortality was assessed at 48 hours.

The following compounds were active at 1lg:-
9, 15, 17, 29, 30, 31, 33, 47.

The activity of compounds of the invention against anaesthetised male Periplaneta americana was demonstrated by the topical application to the test insect of a solution of the compound under test in butanone. Mortality was assessed after 6 days.

The following compounds were active at <50lg:-
2, 6, 16, 17, 31, 39

The activity of compounds of the invention against anaesthetised male Blattella germanica was demonstrated by topical application to the test insect of a solution of the compound under test in butanone. Mortality was assessed after 6 days.

The following compounds were active at <5lg:-
6, 9, 15, 16, 17, 29, 30, 31, 33, 45, 46, 48, 56, 57, 58, 71, 72.

## Nematocidal activity

### Meloidogyne incognita

Selected compounds of the invention were assayed on freshly hatched J2 Meloidogyne incognita. The test solution comprised 1% acetone with 100 p.p.m. Triton X - 100. Activities were assessed after 24 hours.

The following compounds were active at elss than 100 p.p.m.:-
6, 16, 17,

## Mammalian toxicity

The toxicity of compounds of the invention was determined by oral intubation of Charles River CD1 mice, fasted for 3 hours. The compounds were administered as solutions in DMSO at 200mg/10ml/kg, 20mg/10ml/kg and 2mg/10ml/kg. Toxicity was assessed over a 14 day period after dosing.

The following compounds gave an $LD_{50}$ >200mgkg$^{-1}$
17.

The following compounds gave an $LD_{50}$ in the region of 20-200mgkg$^{-1}$
16.

APPENDIX 2

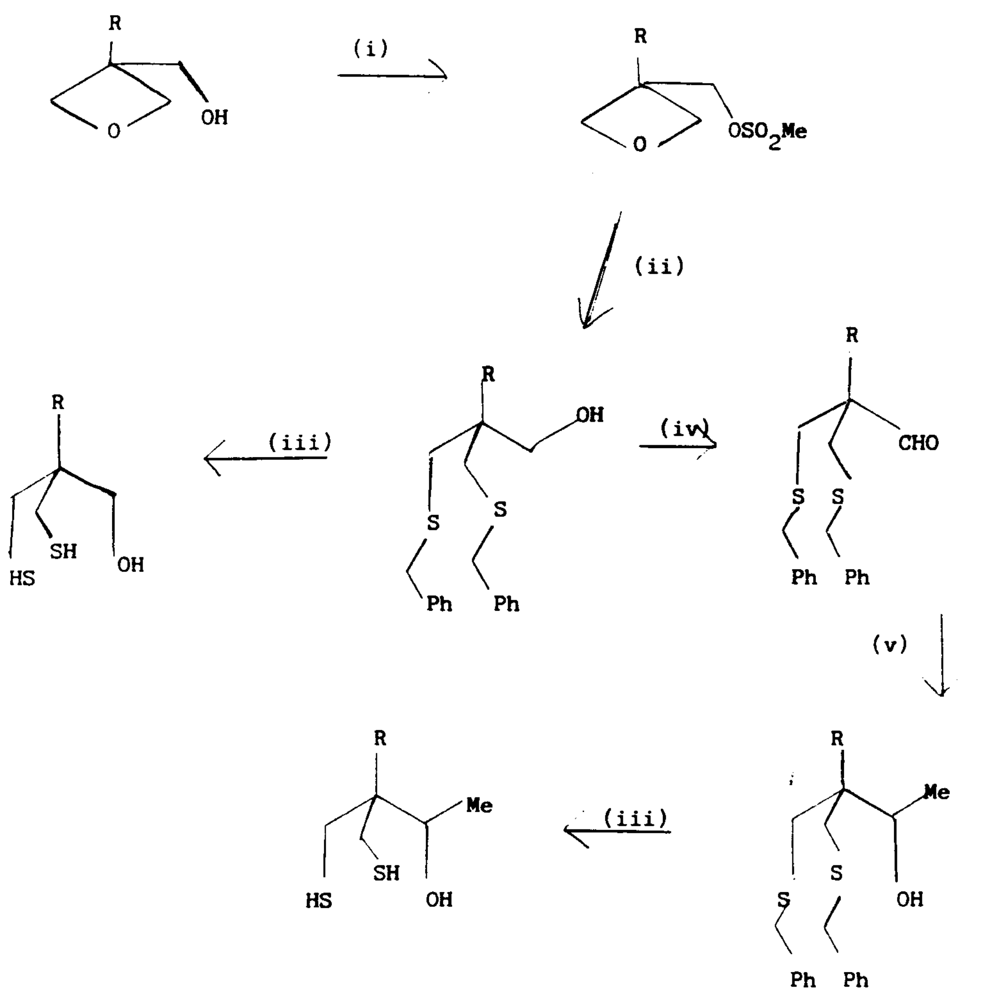


(i) $MeSO_2Cl$, pyridine (ii) $PhCH_2SH$, NaH, Dimethylformamide

(iii) Na, liquid $NH_3$ (iv) Pyridinium chlorochromate, sodium acetate, $CH_2Cl_2$

(v) MeMgI, $Et_2O$

APPENDIX 3

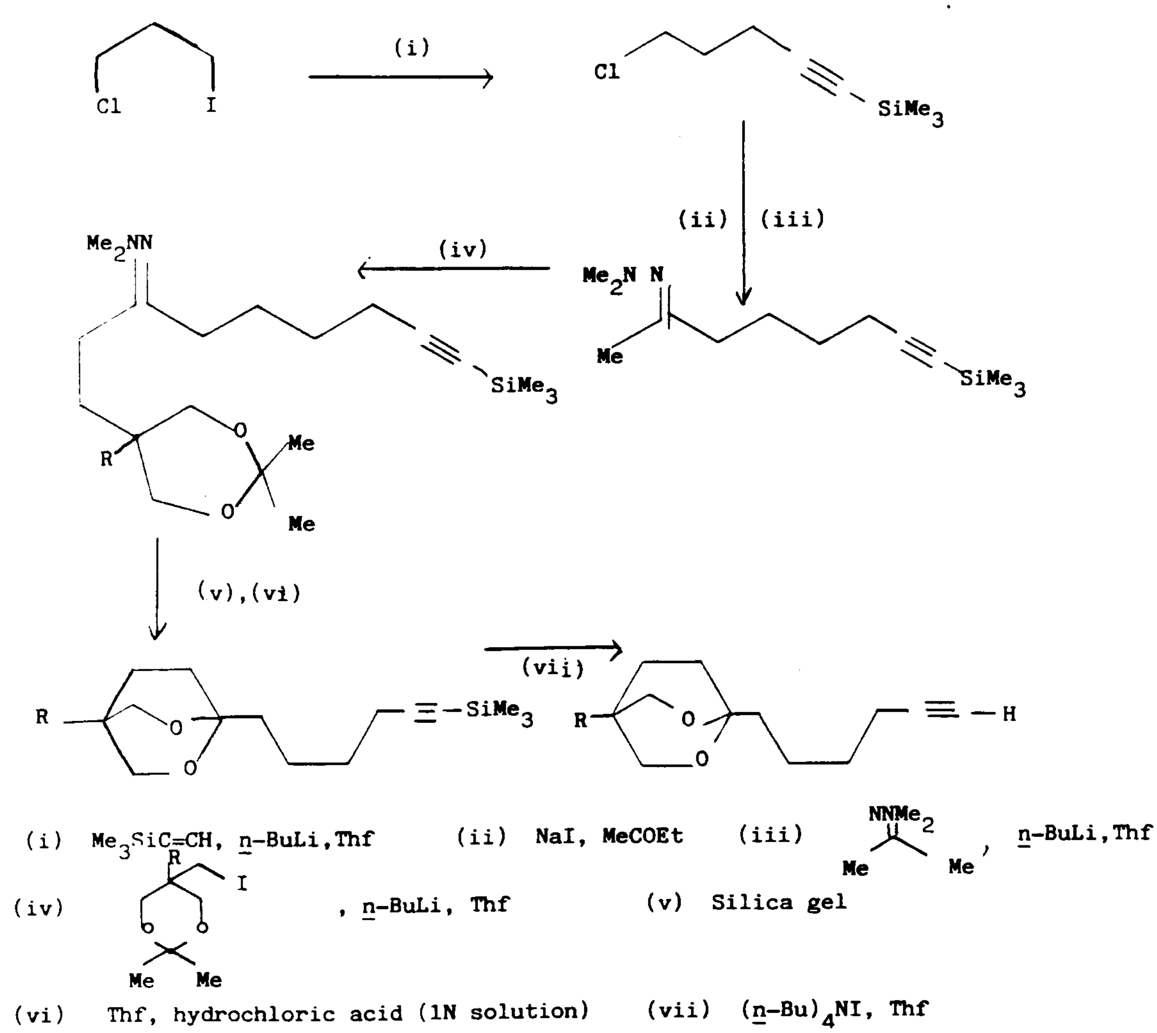
(i)
(ii) (iii)
(iv)
(v),(vi)
(vii)
(i) $Me_3SiC{\equiv}CH$, n-BuLi,Thf
(ii) NaI, MeCOEt
(iii) , n-BuLi,Thf
(iv) , n-BuLi, Thf
(v) Silica gel
(vi) Thf, hydrochloric acid (1N solution)
(vii) $(n\text{-}Bu)_4NI$, Thf

Table I - Trioxabicyclo-octanes

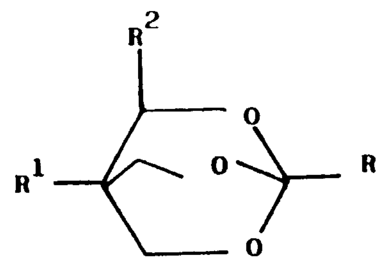

| Compound No. | R | $R^1$ | $R^2$ | Synthetic Method Example |
|---|---|---|---|---|
| 2 | Hex-5-ynyl | n-Pr | H | I |
| 4 | Hex-5-ynyl | n-Pr | $CF_3$ | II |
| 6 | Hex-5-ynyl | n-Pr | CN | III |
| 8 | Hex-5-ynyl | Cyclohexyl | H | III |
| 9 | Hex-5-ynyl | t-Bu | H | IV |

| | | | | |
|---|---|---|---|---|
| 29 | Hex-5-ynyl | n-Bu | CN | III |
| 30 | Hex-5-ynyl | 2-Methylprop-2-enyl | CN | III |
| 31 | Hex-5-ynyl | Prop-2-enyl | CN | XVII |
| 33 | Hex-5-ynyl | But-3-enyl | CN | XVII |

| | | | | |
|---|---|---|---|---|
| 47 | Hex-5-ynyl | <u>t</u>-Bu | CN | IV |
| 48 | Hex-5-ynyl | <u>i</u>-Bu | CN | IV |
| 49 | Hex-5-ynyl | $MeOCH_2$ | CN | X |
| 50 | Hex-5-ynyl | $EtOCH_2$ | CN | X |
| 56 | Hex-5-ynyl | Ph | H | IV |

| | | | | |
|---|---|---|---|---|
| 78 | Hex-5-ynyl | Phenyl | CN | IV |
| 79 | Hex-5-ynyl | Cyclopropyl-methyl | CN | IV |
| 86 | Hex-5-ynyl | 2,2-Dichloro-cyclopropyl-methyl | H | XII |

Table II - Other Bicyclo-octanes

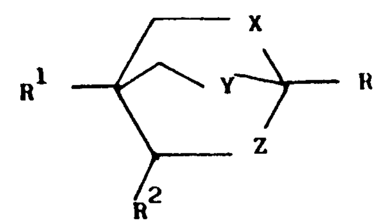

| Compound No. | R | $R^1$ | $R^2$ | X | Y | Z | Synthetic Method Example |
|---|---|---|---|---|---|---|---|
| 15 | Hex-5-ynyl | t-Bu | H | S | O | O | XI |
| 16 | Hex-5-ynyl | n-Pr | H | S | S | O | VI |
| 17 | Hex-5-ynyl | n-Pr | H | S | S | S | VII |
| 45 | Hex-5-ynyl | n-Bu | H | S | S | O | VI |
| 46 | Hex-5-ynyl | t-Bu | H | S | S | O | VI |
| 57 | Hex-5-ynyl | i-Bu | H | S | S | O | VI |
| 58 | Hex-5-ynyl | n-Pr | Me | S | S | O | VIII |
| 71 | Hex-5-ynyl | i-Bu | H | S | S | S | VII |
| 72 | Hex-5-ynyl | Ph | H | S | S | O | VI |
| 73 | Hex-5-ynyl | n-Pr | H | $CH_2$ | O | O | IX |
| 76 | Hex-5-ynyl | Et | H | S | S | S | VII |
| 84 | Hex-5-ynyl | Cyclo-propyl-methyl | H | S | S | O | VI |

Table III - Characterising Data for Trioxabicyclo-octanes

| Compound No. | m.p. | Mass Spectrum Chemical Ionisation M+1 | Nuclear Magnetic Resonance Spectrum $^1H$(ppm from TMS in $CDCl_3$, integral, multiplicity, $^3J_{Hz}$) |
|---|---|---|---|
| 2 | Oil | 239 | 3.90,6H,s;2.17,2H,m;1.95,1H,t; 1.75-1.45,6H,m;1.35-1.05,4H,m; 0.95,3H,t. |
| 4 | 57° | 307 | 4.35,1H,qd;4.15,1H,dd;4.0-3.75,3H, m;2.15,2H,m;1.95,1H,t;1.8-1.1, 10H,m;0.9,3H,t. |
| 6 | Oil | 264 | 4.8,1H,d;4.2,1H, dd;4.05-3.8,3H,m; 2.15,2H,m;1.9,1H,t;1.85-1.1,10H,m; 0.95,3H,t. |

| | | | |
|---|---|---|---|
| 8 | Oil | 279 | 3.95,6H,s;2.2,2H,m;1.95,1H,t; 1.85-0.8,17H,m. |
| 9 | $75^{O}$ | 253 | 4.00,6H,s;2.20,2H,m;1.95,1H,t, 1.70-1.50, 6H, m; 0.85, 9H, s. |
| 10 | $87-90.5^{O}$ | 325 | 4.00, 6H, s; 2.20,2H,m;1.70-1.50, 6H,m;0.85, 9H, s; 0.15, 9H,s. |

| | | | |
|---|---|---|---|
| 29 | Oil | 278 | 4.8,1H,d,2.6;4.2,1H,m;4.05-3.85,3H,m;2.2,2H,m;<br>1.95,1H,t,2.6;1.75-1.15,12H,m;0.9,3H,t,7. |
| 30 | Oil | 276 | 5.05,1H,d;4.8,2H,m;4.23,1H,m;4.1-3.9,3H,m;<br>2.25-2.10,4H,m;1.95,1H,t;1.75,3H,s;<br>1.70-1.65,2H,m;1.60-1.45,4H,m. |
| 31 | Oil | 262 | 5.75-5.5,1H,m;5.20,2H,m;4.75,1H,d;4.20,1H,dd;<br>4.02-3.88,3H,m;2.20-2.12,4H,m;1.95,1H,t;<br>1.75-1.65,2H,m;1.60-1.50,4H,m. |
| | | | d;1.97,1H,t. |
| 33 | Oil | 276 | 5.8-5.65,1H,m;5.06,2H,m;4.80,1H,d;4.20,1H,dd;<br>4.05-3.90,3H,m;2.25-2.15,2H,m;2.07-1.97,2H,m;<br>1.95,1H,t;1.75-1.65,2H,m;1.6-1.45,6H,m. |

| | | | |
|---|---|---|---|
| | | | 2.00,1H,t;0.85,9H,s. |
| 47 | 59° | 278 | 4.80.1H,d;4.35,1H,m;4.00,2H,m;3.85,1H,m; 2.20,2H,m;1.95,1H,t;1.75,2H,m;1.45,4H,m; 1.00,9H,s. |
| 48 | oil | 278 | 4.80,1H,d;4.2-4.9,4H,m;2.2,2H,m;1.9,1H,t; 1.8-1.5,6H,m;1.3,3H,m;1.0,6H,m. |
| 49 | Oil | 266 | 5.00,1H,d;4.30-3.80,6H,m;3.32,3H,s;2.20,2H,m;1.95, 1H,t;1.75,2H,m;1.50,4H,m. |
| 50 | Oil | 280 | 5.02,1H,d;4.20-3.90,4H,m;3.60-3.20,4H,m; 2.20,2H,m;1.95,1H,t;1.70,2H,m;1.53,4H,m; 1.17,3H,t, |
| 56 | 68° | 273 | 7.30,3H,m; 7.15,2H,m; 4.30,6H,s; 2.20,2H,m; 1.95,1H,t; 1.80,2H,m; 1.60,4H,m. |

| | | | |
|---|---|---|---|
| 78 | Oil | 298 | 7.50-7.10,5H,m;5.15,1H,d;4.75,1H,m; 4.35,2H,m; 4.20,1H,d;2.20,2H,m;2.00,1H,t; 1.85,2H,m; 1.60,4H,m. |
| 79 | Oil | 276 | 4.90,1H,d;4.20-3.90,4H,m;2.20,2H,m; 2.00, 1H,t;1.90-1.20,8H,m;0.55,3H,m; 0.15,2H,m. |
| 80 | Oil | 292 | 4.80,1H,d;4.30-3.90,4H,m;2.15,2H,m; 2.00,1H,t;1.80-1.50,5H,m;1.35,3H,m; 0.95,9H,m. |

| | | | |
|---|---|---|---|
| 85 | 51.2° | 253 | 3.90,6H,s;2.15,2H,m;1.75,3H,t;1.65,2H,m;<br>1.50,4H,m;1.20,4H,m;0.90,3H,t. |
| 86 | 80° | 319 | 4.00,6H,s;2.20,2H,m;1.95,1H,t;1.80-1.30, 11H,m. |

Table IV - Characterising Data for Other Bicyclo-octanes

| Compound No. | m.pt. | Mass Spectrum Chemical Ionisation M+1 | Nuclear Magnetic Resonance Spectrum $^1H$(ppm from TMS in $CDCl_3$, integral, multiplicity, $^3J_{Hz}$) |
|---|---|---|---|
| 15 | 68° | 269 | 4.05,4H,s; 3.00,2H,s; 2.20,2H,m; 1.95,1H,t,1.0;1.75,2H,m; 1.55,4H,m; 0.90,9H,s. |
| 16 | 62° | 271 | 4.00,2H,m; 3.00,4H, m; 2.20,2H,m; 2.00,3H,m; 1.55,4H,m; 1.30,4H,m; 0.95,3H,t,7. |
| 17 | 89-92° | 287 | 3.00,6H,s; 2.20,2H,m; 2.00,3H,m; 1.90-1.10,8H,m;0.95,3H,t,7. |
| 45 | Oil | 285 | 4.00,2H,s; 3.05,2H,d; 2.95,2H,d; 2.20,2H,m; 1.92,3H,m; 1.60,4H,m; 1.30,6H,m; 0.90,3H,t,7. |
| 46 | 53° | 285 | 4.10,2H,s; 3.05,4H,dd; 2.18,2H,m; 1.95,3H,m; 1.60,4H,m; 0.95,9H,s. |
| 57 | <30° | 285 | 4.05,2H,s; 3.05,4H,dd; 2.20,2H,m; 2.00,3H,m; 1.75,1H,heptet; 1.55,4H,m; 1.20,2H,d; 1.00,6H,d. |
| 58 | 44° | 285 | 4.25,1H,q; 3.00,4H,m; 2.20,2H,m; 1.95,3H,m; 1.50,4H,m; 1.75,7H,m; 1.00,3H,t. |

| | | | |
|---|---|---|---|
| 71 | 37.1° | 301 | 3.10,6H,s; 2.20,2H,m; 2.00,3H,m; 1.60,5H,m; 1.40,2H,d; 1.00,6H,d. |
| 72 | 70.2° | 305 | 7.35,5H,m; 4.40,2H,s; 3.40,4H,m; 2.20,2H,m; 1.95,3H,m; 1.45,4H,m. |
| 73 | Oil | 237 | 3.8,4H,m; 2.2,2H,m; 2.0-1.9,3H,m; 1.7-1.4,8H,m; 1.3-1.0,4H,m;0.9,3H,t. |
| 84 | Oil | 273 | 3.00,6H,s;2.20,2H,m;2.00,3H,m; 1.90-1.50,6H,m;1.00,3H,t. |
| 89 | Oil | 283 | 4.05,2H,s;3.10,4H,m;2.20,2H,m;2.00,3H,m; 1.60,4H,m;1.25,2H,m;0.60,3H,m;0.10,2H,m. |

## Claims

1. A compound of the formula (I):

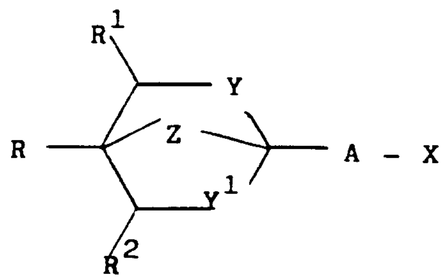

(I)

wherein R is a $C_{2-10}$ non-aromatic hydrocarbyl group optionally substituted by, or methyl substituted by cyano, halo, $C_{1-4}$ alkoxy optionally substituted by halo, or a group $S(O)_mR^3$ where $R^3$ is $C_{1-4}$ alkyl optionally substituted by halo and m is 0, 1 or 2, or R is phenyl optionally substituted by $C_{1-4}$ alkoxy, $C_{1-3}$ alkyl, $C_{2-4}$ alkynyl, halo, $C_{1-4}$ haloalkyl, cyano or a group $S(O)_mR^3$ as defined hereinbefore;
$R^1$ and $R^2$ may be the same or different, and each is hydrogen, methyl $CF_3$ or cyano ;
A-X is $(CH_2)_4$-C≡C-H;
Y and $Y^1$ are the same or different and are each selected from oxygen and $S(O)_{n'}$ where n' is 0, 1 or 2; and Z is $CH_2CH_2$, $CH_2O$ or $CH_2S(O)_{n''}$ wherein n'' is 0, 1 or 2.

2. A compound of the formula (I) according to claim 1 wherein R is n-propyl, n-butyl, i-butyl, t-butyl or phenyl.

3. A compound selected from:
1-(Hex-5-ynyl)-4-propyl-2,6,7-trioxabicyclo[2.2.2]octane
1-(Hex-5-ynyl)-4-propyl-3-trifluoromethyl-2,6,7-trioxabicyclo [2.2.2]octane
1-(Hex-5-ynyl)-4-propyl-2,6,7-trioxabicyclo[2.2.2]octane-3-carbonitrile
4-(Cyclohexyl)-1(hex-5-ynyl)-2,6,7-trioxabicyclo[2.2.2]octane
4-t-Butyl-1-(hex-5-ynyl)-2,6,7-trioxabicyclo[2.2.2]octane
4-t-Butyl-1-(hex-5-ynyl)-2,6-dioxa-7-thiabicyclo[2.2.2]octane
1-(Hex-5-ynyl)-4-propyl-2-oxa-6,7-dithiabicyclo[2.2.2]octane
1-(Hex-5-ynyl)-4-propyl-2,6,7-trithiabicyclo[2.2.2]octane
4-Butyl-1-(hex-5-ynyl)-2,6,7-trioxabicyclo[2.2.2]octane-3-carbonitrile
1-(Hex-5-ynyl)-4-(2-methylprop-2-enyl)-2,6,7-trioxabicyclo[2.2.2] octane-3-carbonitrile
1-(Hex-5-ynyl)-4-(prop-2-enyl)-2,6,7-trioxabicyclo[2.2.2]octane-3-carbonitrile
4-(But-3-enyl)-1-(hex-5-ynyl)-2,6,7-trioxabicyclo[2.2.2]octane-3-carbonitrile
4-Butyl-1-(hex-5-ynyl)-2-oxa-6,7-dithiabicyclo[2.2.2]octane
4-t-Butyl-1-(hex-5-ynyl)-2-oxa-6,7-dithiabicyclo[2.2.2]octane
4-t-Butyl-1-(hex-5-ynyl)-2,6,7-trioxabicyclo[2.2.2]octane-3-carbonitrile
1-(Hex-5-ynyl)-4-isobutyl-2,6,7-trioxabicyclo[2.2.2]octane-3-carbonitrile
4-Ethoxymethyl-1-(hex-5-ynyl)-2,6,7-trioxabicyclo(2.2.2]octane-3-carbonitrile
1-(Hex-5-ynyl)-4-phenyl-2,6,7-trioxabicyclo[2.2.2]octane
1-(Hex-5-ynyl)-4-isobutyl-2-oxa-6,7-dithiabicyclo[2.2.2]octane
1-(Hex-5-ynyl)-3-methyl-4-propyl-2-oxa-6,7-dithiabicyclo[2.2.2] octane
1-(Hex-5-ynyl)-4-isobutyl-2,6,7-trithiabicyclo[2.2.2]octane
1-(Hex-5-ynyl)-4-phenyl-2-oxa-6,7-dithiabicyclo[2.2.2]octane
4-Ethyl-1-(hex-5-ynyl)-2,6,7-trithiabicyclo[2.2.2]octane
1-(Hex-5-ynyl)-4-phenyl-2,6,7-trioxabicyclo[2.2.2]octane-3-carbonitrile
4-Cyclopropylmethyl-1-(hex-5-ynyl)-2,6,7-trioxabicyclo[2.2.2]octane-3-carbonitrile

4. A pesticidal formulation comprising a compound of the formula (I) as defined according to any of the preceding claims in admixture with one or more carriers or diluents.

5. A pesticidal formulation according to claim 4 which additionally contains a synergist or potentiator.

**6.** A pesticidal formulation according to either claim 4 or claim 5 which additionally contains one or more pesticidally active ingredients, attractants, repellents, bacteriocides, fungicides and/or anthelmintics.

**7.** A compound of the formula (I) for use in human or veterinary medicine.

**8.** A method for the control of arthropod or helminth pests which comprises administering to the arthropod or helminth or their environment an effective amount of a compound of the formula (I) as prepared according to any one of the preceding claims.

**9.** A method for the control of pesticidal infestations on plants and/or stored products and/or an environment which comprises administering an effective amount of a compound of the formula (I) as defined according to any one of the preceding claims to the plant and/or stored product and/or an environment susceptible to pest infestation.

**10.** A process for the manufacture of a compound of the formula (I) as defined according to claim 1 which comprises:

i) for the production of compounds wherein Y and $Y^1$ are oxygen and Z is $CH_2O$:

a) the cyclisation of a compound of the formula (II):

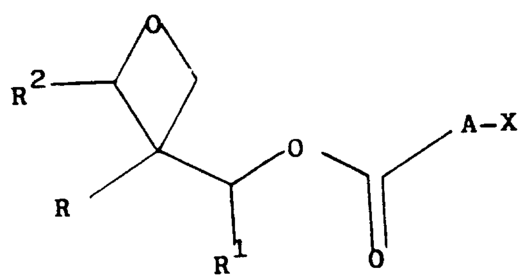

(II)

wherein R to $R^2$, A and X are as defined according to claim 1, in the presence of an acid catalyst or;

b) the reaction of a strong base with a compound of the formula (VII):

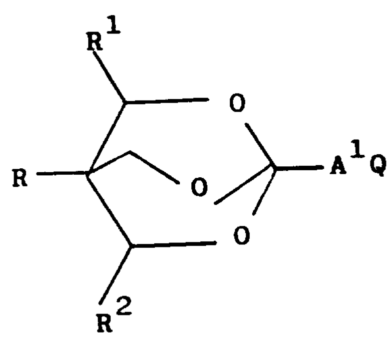

(VII)

wherein R to $R^2$ and A are as defined according to claim 1 and Q is a group capable of conversion into an ethynyl group or;

ii) for the production of compounds

wherein n' is O, $Y^1$ is O or S, Y is O or S, Z is $CH_2S$ or $CH_2O$, the reaction of a compound $(AlkO)_3C$ CAX with a compound of the formula (XIV):

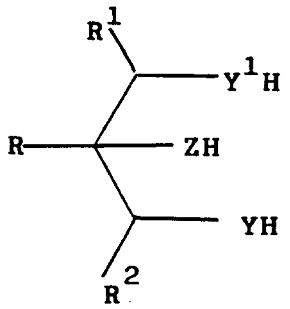

(XIV)

wherein R to $R^2$, A, X, Y, $Y^1$ and Z are as defined according to claim 1 and Alk is a $C_{1-4}$ alkyl group or;

iii) for the production of compounds wherein $Z = CH_2S$ or $CH_2O$ and Y and $Y^1$ are sulphur the reaction of a compound $L^2AX$ with a compound of the formula (XV):

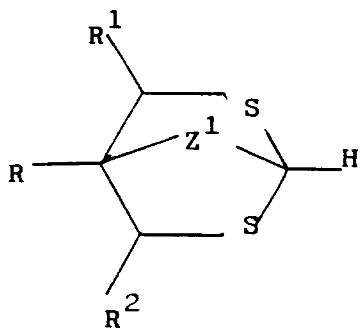

(XV)

**Patentansprüche**

**1.** Verbindung der Formel (I):

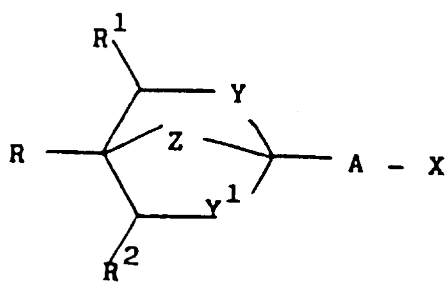

(I)

worin R eine $C_{2-10}$-nichtaromatische Kohlenwasserstoffgruppe, gegebenenfalls substituiert mit, oder Methyl, substituiert mit Cyano, Halogen, $C_{1-4}$-Alkoxy, gegebenenfalls substituiert mit Halogen, oder einer Gruppe $S(O)_mR^3$, worin $R^3$ $C_{1-4}$-Alkyl, gegebenenfalls substituiert mit Halogen, bedeutet und m 0, 1 oder 2 ist, darstellt, oder R Phenyl, gegebenenfalls substituiert mit $C_{1-4}$-Alkoxy, $C_{1-3}$-Alkyl, $C_{2-4}$-Alkinyl, Halogen, $C_{1-4}$-Halogenalkyl, Cyano oder einer Gruppe $S(O)_mR^3$, wie hierin vorstehend definiert, ist;

$R^1$ und $R^2$ gleich oder verschieden sein können, und jeweils Wasserstoff, Methyl, $CF_3$ oder Cyano sind;

A-X $(CH_2)_4$-C≡C-H ist;

Y und $Y^1$ gleich oder verschieden sind und jeweils ausgewählt sind aus Sauerstoff und $S(O)_{n'}$, worin n' 0, 1 oder 2 ist; und Z $CH_2CH_2$, $CH_2O$ oder $CH_2S(O)_{n''}$ bedeutet, worin n'' 0, 1 oder 2 ist.

**2.** Verbindung der Formel (I) nach Anspruch 1, wobei R n-Propyl, n-Butyl, i-Butyl, t-Butyl oder Phenyl darstellt.

**3.** Verbindung, ausgewählt aus:

1-(Hex-5-inyl)-4-propyl-2,6,7-trioxabicyclo[2.2.2]octan
1-(Hex-5-inyl)-4-propyl-3-trifluormethyl-2,6,7-trioxabicyclo[2.2.2]octan
1-(Hex-5-inyl)-4-propyl-2,6,7-trioxabicyclo[2.2.2]octan-3-carbonitril
4-(Cyclohexyl)-1-(hex-5-inyl)-2,6,7-trioxabicyclo[2.2.2]octan
4-t-Butyl-1-(hex-5-inyl)-2,6,7-trioxabicyclo[2.2.2]-octan
4-t-Butyl-1-(hex-5-inyl)-2,6-dioxa-7-thiabicyclo[2.2.2]octan
1-(Hex-5-inyl)-4-propyl-2-oxa-6,7-dithiabicyclo[2.2.2]octan
1-(Hex-5-inyl)-4-propyl-2,6,7-trithiabicyclo[2.2.2]octan
4-Butyl-1-(hex-5-inyl)-2,6,7-trioxabicyclo[2.2.2]octan-3-carbonitril
1-(Hex-5-inyl)-4-(2-methylprop-2-enyl)-2,6,7-trioxabicyclo[2.2.2]octan-3-carbonitril
1-(Hex-5-inyl)-4-(prop-2-enyl)-2,6,7-trioxabicyclo[2.2.2]octan-3-carbonitril
4-(But-3-enyl)-1-(hex-5-inyl)-2,6,7-trioxabicyclo[2.2.2]octan-3-carbonitril
4-Butyl-1-(hex-5-inyl)-2-oxa-6,7-dithiabicyclo[2.2.2]octan
4-t-Butyl-1-(hex-5-inyl)-2-oxa-6,7-dithiabicyclo[2.2.2]octan
4-t-Butyl-1-(hex-5-inyl)-2,6,7-trioxabicyclo[2.2.2]octan-3-carbonitril
1-(Hex-5-inyl)-4-isobutyl-2,6,7-trioxabicyclo[2.2.2]octan-3-carbonitril
4-Ethoxymethyl-1-(hex-5-inyl)-2,6,7-trioxabicyclo[2.2.2]octan-3-carbonitril
1-(Hex-5-inyl)-4-phenyl-2,6,7-trioxabicyclo[2.2.2]octan
1-(Hex-5-inyl)-4-isobutyl-2-oxa-6,7-dithiabicyclo[2.2.2]octan
1-(Hex-5-inyl)-3-methyl-4-propyl-2-oxa-6,7-dithiabicyclo[2.2.2]octan
1-(Hex-5-inyl)-4-isobutyl-2,6,7-trithiabicyclo[2.2.2]octan
1-(Hex-5-inyl)-4-phenyl-2-oxa-6,7-dithiabicyclo[2.2.2]octan
4-Ethyl-1-(hex-5-inyl)-2,6,7-trithiabicyclo[2.2.2]octan
1-(Hex-5-inyl)-4-phenyl-2,6,7-trioxabicyclo[2.2.2]octan-3-carbonitril
4-Cyclopropylmethyl-1-(hex-5-inyl)-2,6,7-trioxabicyclo[2.2.2]octan-3-carbonitril

**4.** Pestizidformulierung, umfassend eine wie in einem der vorangehenden Ansprüchen definierte Verbindung der Formel (I) in Anmischung mit einem oder mehreren Trägern oder Verdünnungsmitteln.

**5.** Pestizidformulierung nach Anspruch 4, die zusätzlich einen Synergisten oder Potentiator enthält.

**6.** Pestizidformulierung nach entweder Anspruch 4 oder Anspruch 5, die zusätzlich einen oder mehrere pestizide Wirkstoffe, Lockmittel, Abwehrmittel, Bakterizide, Fungizide und/oder Anthelmintika enthält.

**7.** Verbindung der Formel (I) zur Verwendung in der Human- oder Veterinärmedizin.

**8.** Verfahren zur Bekämpfung von arthropoden oder helminthischen Schädlingen, umfassend Verabreichen an den Gliederfüßer oder den Eingeweidewurm oder deren Umgebung in einer wirksamen Menge einer gemäß einer der vorangehenden Ansprüche hergestellten Verbindung der Formel (I).

**9.** Verfahren zur Bekämpfung von Schädlingsbefall auf Pflanzen und/oder gelagerten Produkten und/oder in eine Umgebung, umfassend Verabreichen einer wirksamen Menge einer nach einem der vorangehenden Ansprüche definierten Verbindung der Formel (I), auf die Pflanzen und/oder das gelagerte Produkt und/oder eine Umgebung, die empfänglich für Schädlingsbefall sind.

**10.** Verfahren zur Herstellung einer wie in Anspruch 1 definierten Verbindung der Formel (I), umfassend:

i) zur Herstellung von Verbindungen, worin Y und $Y^1$ Sauerstoff darstellen und Z $CH_2O$ bedeutet:

a) Cyclisierung einer Verbindung der Formel (II):

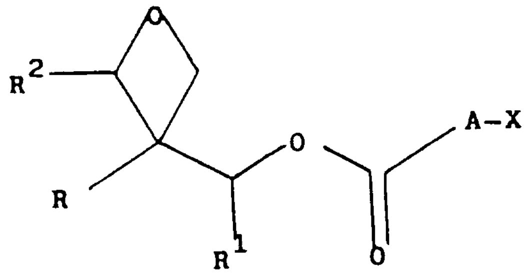

(II)

worin R bis $R^2$, A und X wie vorstehend in Anspruch 1 definiert sind, in Gegenwart eines sauren Katalysators oder;

b) Umsetzung einer starken Base mit einer Verbindung der Formel (VII):

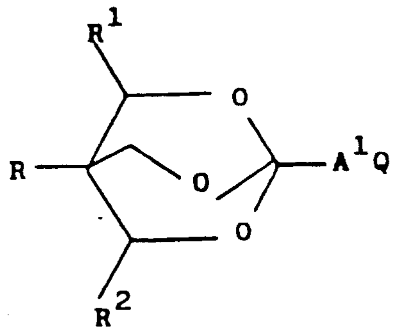

(VII)

worin R bis $R^2$ und A wie vorstehend in Anspruch 1 definiert sind und Q eine Gruppe, geeignet zur Umwandlung in eine Ethinylgruppe ist, oder

ii) zur Herstellung von Verbindungen,

worin n' O ist, $Y^1$ O oder S ist, Y O oder S ist, Z $CH_2S$ oder $CH_2O$ ist, Umsetzung einer Verbindung $(AlkO)_3$ CAX mit einer Verbindung der Formel (XIV):

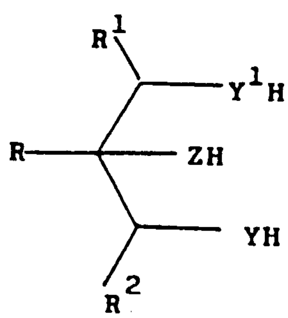

(XIV)

worin R bis $R^2$, A, X, Y, $Y^1$ und Z wie in Anspruch 1 definiert sind und Alk eine $C_{1-4}$-Alkylgruppe darstellt oder;

iii) zur Herstellung von Verbindungen, worin Z = $CH_2S$ oder $CH_2O$ und Y und $Y^1$ Schwefel sind, Umsetzung einer Verbindung $L^2AX$ mit einer Verbindung der Formel (XV):

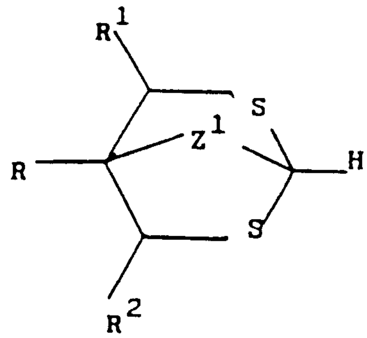

(XV)

## Revendications

**1.** Composé de formule (I):

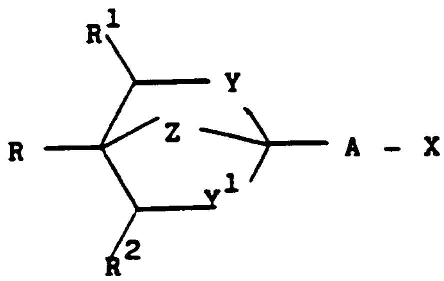

(I)

dans laquelle R est un groupement hydrocarbyle non-aromatique en $C_{2-10}$ éventuellement substitué par, ou méthyl-substitué par cyano, halogéno, alcoxy en $C_{1-4}$ éventuellement substitué par halogéno, ou un groupement $S(O)_mR^3$ où $R^3$ est alkyl en $C_{1-4}$ éventuellement substitué par halogéno et m est 0, 1 ou 2, ou R est phényle éventuellement substitué par alcoxy en $C_{1-4}$, alkyle en $C_{1-3}$, alkynyle en $C_{2-4}$, halogéno, halogénoalkyle en $C_{1-4}$, cyano ou un groupement $S(O)_mR^3$ comme défini précédemment;

$R^1$ et $R^2$ peuvent être identiques ou différents, et sont chacun hydrogène, méthyle $CF_3$ ou cyano;

A-X est $(CH_2)_4$-C≡C-H;

Y et $Y^1$ sont identiques ou différents et sont choisis chacun parmi l'oxygène et $S(O)_{n'}$ où n' est 0, 1 ou 2; et Z est $CH_2CH_2$, $CH_2O$ ou $CH_2S(O)_{n''}$ où n'' est 0, 1 ou 2.

**2.** Composé de formule (I) selon la revendication 1, dans laquelle R est n-propyle, n-butyle, i-butyle, t-butyle ou phényle.

**3.** Composé choisi parmi:

1-(Hex-5-ynyl)-4-propyl-2,6,7-trioxabicyclo[2,2,2]octane
1-(Hex-5-ynyl)-4-propyl-3-trifluorométhyl-2,6,7-trioxahicyclo[2,2,2]octane
1-(Hex-5-ynyl)-4-propyl-2,6,7-trioxabicyclo[2,2,2]octane-3-carbonitrile
4-(Cyclohexyl)-1-(hex-5-ynyl)-2,6,7-trioxabicyclo[2,2,2]octane
4-t-Butyl-1-(hex-5-ynyl)-2,6,7-trioxabicyclo[2,2,2]octane
4-t-Butyl-1-(hex-5-ynyl)-2,6-dioxa-7-thiabicyclo[2,2,2]octane
1-(Hex-5-ynyl)-4-propyl-2-oxa-6,7-dithiabicyclo[2,2,2]octane
1-(Hex-5-ynyl)-4-propyl-2,6,7-trithiabicyclo[2,2,2]octane
4-Butyl-1-(hex-5-ynyl)-2,6,7-trioxabicyclo[2,2,2]octane-3-carbonitrile
1-(Hex-5-ynyl)-4-(2-méthylprop-2-ényl)-2,6,7-trioxabicyclo[2,2,2]octane-3-carbonitrile
1-(Hex-5-ynyl)-4-(prop-2-ényl)-2,6,7-trioxabicyclo[2,2,2]octane-3-carbonitrile
4-(But-3-ényl)-1-(hex-5-ynyl)-2,6,7-trioxabicyclo[2,2,2]octane-3-carbonitrile
4-Butyl-1-(hex-5-ynyl)-2-oxa-6,7-dithiabicyclo[2,2,2]octane
4-t-Butyl-1-(hex-5-ynyl)-2-oxa-6,7-dithiabicyclo[2,2,2]octane
4-t-Butyl-1-(hex-5-ynyl)-2,6,7-trioxahicyclo[2,2,2]octane-3-carbonitrile

1-(Hex-5-ynyl)-4-isobutyl-2,6,7-trioxabicyclo[2,2,2]octane-3-carbonitrile
4-Ethoxyméthyl-1-(hex-5-ynyl)-2,6,7-trioxabicyclo[2,2,2]octane-3-carbonitrile
1-(Hex-5-ynyl)-4-phényl-2,6,7-trioxabicyclo[2,2,2]octane
1-(Hex-5-ynyl)-4-isobutyl-2-oxa-6,7-dithiabicyclo[2,2,2]octane
1-(Hex-5-ynyl)-3-méthyl-4-propyl-2-oxa-6,7-dithiabicyclo[2,2,2]octane
1-(Hex-5-ynyl)-4-isobutyl-2,6,7-trithiabicyclo[2,2,2]octane
1-(Hex-5-ynyl)-4-phényl-2-oxa-6,7-dithiabicyclo[2,2,2]octane
4-Ethyl-1-(hex-5-ynyl)-2,6,7-trithiabicyclo[2,2,2]octane
1-(Hex-5-ynyl)-4-phényl-2,6,7-trioxabicyclo[2,2,2]octane-3-carbonitrile
4-Cyclopropylméthyl-1-(hex-5-ynyl)-2,6,7-trioxabicyclo[2,2,2]octane-3-carbonitrile

**4.** Formulation pesticide comprenant un composé de formule (I) tel que défini selon l'une quelconque des revendications précédentes, en mélange avec un ou plusieurs supports ou diluants.

**5.** Formulation pesticide selon la revendication 4, caractérisée en ce qu'elle contient en outre un synergiste ou un potentialisateur.

**6.** Formulation pesticide selon l'une quelconque des revendications 4 ou 5, caractérisée en ce qu'elle contient en outre un ou plusieurs ingrédients, attractifs, répulsifs, bactéricides, fongicides et/ou anthelminthiques actifs du point de vue pesticide.

**7.** Composé de formule (I), destiné à être utilisé en médecine humaine ou vétérinaire.

**8.** Méthode de lutte contre les arthropodes ou les helminthes nuisibles comprenant l'administration à l'arthropode ou à l'helminthe ou à leur environnement une quantité efficace d'un composé de formule (I) tel que préparé selon l'une quelconque des revendications précédentes.

**9.** Méthode de lutte contre l'infestation de plantes et/ou de produits stockés et/ou d'un environnement à l'aide de pesticides, comprenant l'administration d'une quantité efficace d'un composé de formule (I) tel que défini selon l'une quelconque des revendications précédentes à la plante et/ou au produit stocké et/ou à un environnement susceptible d'être infesté par des organismes nuisibles.

**10.** Procédé de fabrication d'un composé de formule (I) tel que défini selon la revendication 1, caractérisé en ce qu'il comprend:

i) pour la production de composés dans lesquels Y et $Y^1$ sont l'oxygène et Z est $CH_2O$:

a) la cyclisation d'un composé de formule (II):

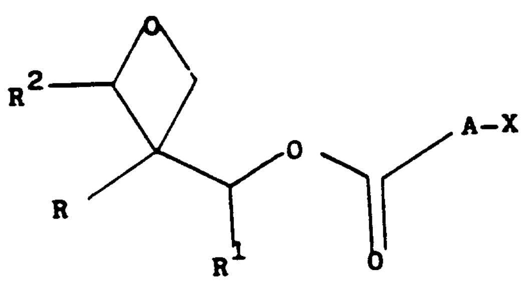

(II)

dans laquelle R à $R^2$, A et X sont tels que définis selon la revendication 1, en présence d'un catalyseur acide; ou

b) la réaction d'une base forte avec un composé de formule (VII):

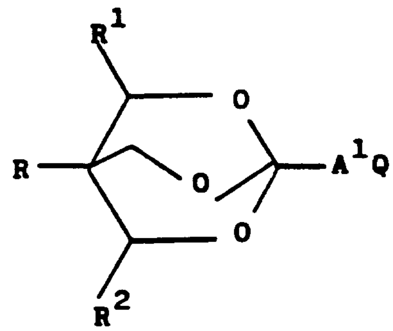

(VII)

dans laquelle R à $R^2$ et A sont tels que définis selon la revendication 1 et Q est un groupement transformable en un groupement éthynyle; ou

ii) pour la production de composés

dans lesquels n' est 0, $Y^1$ est O ou S, Y est O ou S, Z est $CH_2S$ ou $CH_2O$, la réaction d'un composé $(AlkO)_3C$ CAX avec un composé de formule (XIV):

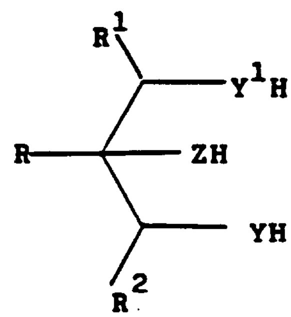

(XIV)

dans laquelle R à $R^2$, A, X, Y, $Y^1$ et Z sont tels que définis selon la revendication 1 et Alk est un groupement alkyle en $C_{1-4}$; ou

(iii) pour la production de composés dans lesquels $Z \equiv CH_2S$ ou $CH_2O$ et Y et $Y^1$ sont le soufre, la réaction d'un composé $L^2AX$ avec un composé de formule (XV):

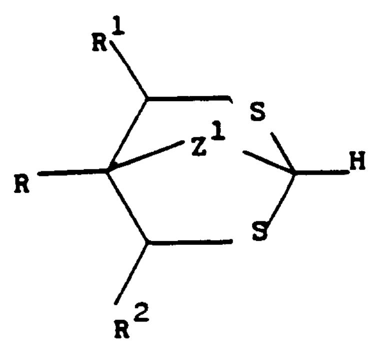

(XV)